# EUROPEAN PATENT SPECIFICATION

(11) **EP 3 048 971 B1**
(45) Date of publication and mention of the grant of the patent: **07.12.2022**
(21) Application number: 14849496.6
(22) Date of filing: 25.08.2014
(51) Int. Cl.: G01N 33/00, A61B 5/08, G01N 33/497

(54) **REMOTE BREATH ALCOHOL MONITORING**
ENTFERNTE ATEMALKOHOLÜBERWACHUNG
SURVEILLANCE À DISTANCE D'ALCOOL DANS L'AIR EXPIRÉ

(30) Priority: 26.09.2013 US 201314038448
(43) Date of publication of application: 03.08.2016
(73) Proprietor: Alcohol Monitoring Systems, Inc., Littleton, CO 80120 (US)
(72) Inventor: WOJCIK, Mark Henry, Littleton, CO 80127 (US); SHOFFNER, Gary Alan, Centennial, CO 80122 (US); MURRAY, Gordon William, Lone Tree, CO 80124 (US); TUBB, Glenn Charles, Denver, CO 80210 (US)
(74) Representative: Lucas, Phillip Brian
(86) International application number: PCT/US2014/052465
(87) International publication number: WO 2015/047618

(56) References cited:
- JP-A- 2009 064 139
- US-A1- 2006 202 837
- US-A1- 2009 293 589
- US-A1- 2010 108 425
- US-A1- 2011 079 073
- US-A1- 2012 075 462
- US-A1- 2012 188 532
- US-A1- 2013 035 602
- US-A1- 2013 035 602
- US-A1- 2013 245 483
- Hyung-Keun Jee ET AL: "Liveness Detection for Embedded Face Recognition System", World Academy of Science, Engineering and Technology 18, 1 January 2008 (2008-01-01), XP055359123, Retrieved from the Internet: URL:http://citeseerx.ist.psu.edu/viewdoc/d ownload?doi=10.1.1.307.9254&rep=rep1&type= pdf [retrieved on 2017-03-27]

## Description

### BACKGROUND

This application relates to the technical field of breath alcohol monitoring, and more particularly to a portable handheld wireless breath alcohol monitoring device that utilizes facial recognition and automatic retesting if an initial test is positive for alcohol or if an initial facial match is negative. A particular concern of this invention is to prevent an offender from circumventing a test.

When an offender is convicted of DUI, domestic violence, or another alcohol-related offense, a typical condition of sentencing or probation is that the individual must stop drinking for a specified period of time. To enforce this prohibition, courts have traditionally looked to random testing methods (blood, breath, ethyl glucuronide or EtG) that only showed if the individual was sober at the specific "point in time" the test was administered. Requiring sobriety of substance-involved offenders under correctional supervision has been trending since the 1980s, when the first Drug Court was established in Miami-Dade County, Florida. Over the next twenty-five years, enforcing sobriety for drug offenders became standard operating procedure in programs throughout the country. Random drug testing became the cornerstone of these treatment-focused courts. The overall philosophy of jurisprudence shifted to a focus on treating the addiction and utilizing swift interventions and sanctions for violations.

Alcohol, however, has proven to be more complex than drugs for the courts to manage. Despite the emphasis on drug abuse, alcohol remains the number one drug of abuse in the U.S. corrections system. In fact, it is widely reported that once drug offenders begin random drug testing, they often switch to alcohol as their drug of choice because alcohol may be legally purchased and with standard, random testing the offender can begin drinking right after a test and sober up before the next test.

While drug testing, which is usually done on a random schedule, is relatively accurate and cost-effective, the metabolism of the human body makes monitoring for alcohol far more complex. Alcohol is metabolized in the liver, which eliminates 95-98% of ingested alcohol from the body. No matter the rate of ingestion, it can only be metabolized at a certain rate, which can vary from person to person. A small amount of alcohol, about 1-5%, avoids metabolism in the liver and is excreted, unchanged, through the kidneys (urine), the lungs (breath), or the skin (perspiration).

Healthy people, on average, metabolize alcohol at a fairly consistent rate: one standard drink (or 0.5 ounces [14.17 grams] of alcohol) per hour. Heavy alcohol users may metabolize alcohol at a significantly higher rate than average individuals.

The result is that it is not just possible, it is probable, that an individual can be tested in the early evening at 6:00 pm or 7:00 pm and then get very intoxicated when they go to bed at 10:00 pm, yet be completely sober in less than eight hours for their next alcohol test. The more severe the alcohol dependence, the faster an individual may metabolize the alcohol and avoid detection between test times.

Breath, blood, and urine testing are all reliable at testing individuals for alcohol consumption at any given "point in time." In 2003 transdermal alcohol testing was introduced as a way to test offenders for alcohol, without requiring active participation of the offender, and at a frequency rate high enough to ensure the offender stayed sober all day long. Typically, for transdermal alcohol testing, an ankle bracelet is attached to the offender with a durable and tamper-proof strap. The ankle bracelet is worn 24/7 by the offender for the duration of his or her court-ordered abstinence period. Periodically, such as every half-hour or hour, the bracelet analyzes samples of the insensible perspiration coming off the offender's skin and generates transdermal alcohol readings. The bracelet stores this data and, at pre-determined times, transmits the data to a base station or a monitoring network where the data can be analyzed. The testing protocol is prescheduled and automated, eliminating the offender's ability to manipulate the testing schedule or avoid or delay a request to test. Transdermal analysis and continuous alcohol monitoring (CAM) weren't developed because conventional testing is unreliable. They were developed because offenders who misuse alcohol are unreliable.

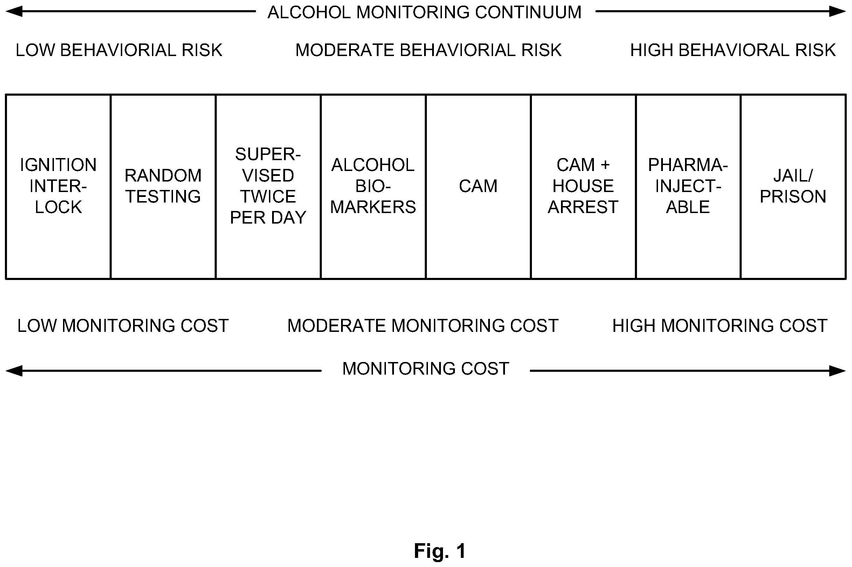

Current testing options to enforce sobriety are available on a continuum, from incarceration-the most intense sanction and most costly per day-to ignition interlock, which when installed only tests for sobriety when someone is driving (see FIG. 1).

These testing options range in cost and vary in terms of behavioral risk. Employing an assessment process to determine how to balance supervision and monitoring costs with the risk level of each offender is essential to a successful alcohol testing and monitoring program. Lower risk offenders who misuse alcohol may be assigned a less intrusive and less expensive testing and monitoring approach. High risk offenders who are alcoholic dependent or addicted may be assigned a more intrusive, vigorous, and expensive testing and monitoring regimen. Upon successful performance over a several month period of time, high risk offenders may be rewarded for their good behavior by being transitioned to a more convenient, less intrusive, and less expensive testing and monitoring approach.

US 2013/0035602 describes a monitoring apparatus including an ethanol level sensor; an ECG sensor for generating an ECG circuit for sampling an electrical activity of a heart of a person being monitored;and an air inlet component connected to an airflow sensor; wherein a first electrode is connected to the breath inlet component and wherein the ECG circuit is generated when a monitored personis in electrical contact with the first electrode and the second electrode of the ECG.

US 2011/009073 describes a hand-held device for monitoring sobriety and constructed to receive a sample of a user's breath and transmit to a receiving station both a signal relating to the breath content and a signal relating to user identification.

The paper "Liveness Detection for Embedded Face Recognition System" by Hyung-Keun Jee et al is published in World Academy of Science, Engineering and Technology 18, published 1st January 2008, XP055359123.

### SUMMARY

This Summary is provided to introduce in a simplified form a selection of concepts that are further described below in the Detailed Description. This Summary is not intended to identify key or essential features of the claimed subject matter, nor is it intended to be used to limit the scope of the claimed subject matter.

The detailed description below describes a portable handheld wireless breath alcohol monitoring device that utilizes facial recognition and automatic retesting if an initial test is positive for alcohol or a negative facial match. In addition, a location fix is captured with each breath test. The solution described below enables the monitoring of lower-risk offenders or offenders who have earned the privilege of a less intrusive alcohol testing and monitoring program.

The remote breath alcohol monitor (RBAM) described below is portable, easy to carry, and easy to use. It can be programmed with flexible testing schedules with multiple options for both the agencies that require the use of the device and for the offenders who are required to use the device. RBAM features an automated intelligent bio-confirmation system that utilizes facial recognition software that automatically matches an enrollment image of the offender with an image taken at the time of a breath test through a built-in high resolution camera, ensuring that the offender being monitored is the individual actually taking the breath test. The facial recognition software is also able to distinguish between a live person and a printed photograph or mask. Other image-based bio-confirmation methods could be adapted to perform the same function, such as iris recognition and cornea recognition. RBAM has built in wireless cellular communication and in one embodiment is GPS enabled to identify a location fix for each test. Other methods may be used to provide a location fix, including cell-tower triangulation, nearest cell site, other network-based or SIM-based methods commonly referred to as location-based services. Other methods being developed includes crowd sourced Wi-Fi data, Wi-Fi positioning systems, and IP-based geo-location methods. RBAM employs an ethanol fuel cell to determine breath alcohol content (BrAC) and can be programmed for scheduled, random, flexible, or on-demand testing. Other types of ethanol sensors that are not fuel cells may also be used, such as metal oxide sensors or proton exchange membranes. It is anticipated that technologies currently under development and future technologies, such as quartz microbalance (QMB) sensors and thin-film bulk acoustic resonator-based (FBAR) sensors technology, may provide still other types of sensors suitable for this purpose. A supervising agency can be immediately notified, or on a priority notification basis within a relatively short period of time, such as fifteen minutes, upon a positive breath test, negative facial match, or other criteria or combinations of criteria. A positive initial test is automatically followed by a retest, or confirmation test. A negative facial match may also be followed by a retest.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xm, Y1-Yn, and Z1-Zo, the phrase is intended to refer to a single element selected from X, Y, and Z, a combination of elements selected from the same class (e.g., X1 and X2) as well as a combination of elements selected from two or more classes (e.g., Y1 and Z3).

It is to be noted that the term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

"Lower Limit of Detection" (LLOD) means a user settable field that may be pulled down from a monitor network by the RBAM or programmed into the RBAM. In one embodiment the proposed range is from 0.020% to 0.050% BrAC in increments of 0.005%, with a default of 0.020%. One skilled in the art will recognize that other units of measure, ranges, increments, and defaults may be used to suit particular demands or purposes. Breath tests results are considered positive (see definition below) if they are ≥ LLOD, and are considered negative (see definition below) if they are < LLOD.

"Valid Sample" means the offender properly blows into the RBAM and provides an adequate breath sample for analysis. All valid samples lead to either a positive or negative breath test result.

"Invalid Sample" means the offender does not properly blow into the RBAM and does not provide a valid breath sample for analysis. This is almost always because the offender did not blow long enough or hard enough. An invalid sample cannot produce a test result.

"No Sample" means the offender does not blow into the RBAM at all. A no sample cannot produce a breath test result.

"Initial Test" means the first test provided at a scheduled test time or an on-demand test. If the initial test is a negative test (see definition below), then it is the only test for that scheduled time or on-demand request.

"Confirmation Test" or "Retest" means a second test taken shortly after an initial test that was a positive test (see definition below) or after a negative facial match. The confirmation test is intended to determine if the initial test was caused by actual deep-lung breath alcohol (and therefore blood alcohol), or by mouth alcohol. To allow time for mouth alcohol to dissipate, the confirmation test occurs after a certain Wait-To-Retest Period (see definition below) has passed. A confirmation test can also be performed after a negative facial match, allowing the offender to remove sunglasses or be given a second chance if they allowed a different person to take their test, for example.

"Retry" means that when there is an invalid sample, the offender is prompted to blow again in order to complete an initial test or a confirmation test. A retry should not be confused with a retest/confirmation test. A confirmation test can also be performed after a negative facial match, allowing the offender to remove sunglasses or be given a second chance if they allowed a different person to take their test, as examples.

"On-Demand Test" means a breath test that occurs outside of the defined test schedule. Personnel from a supervising agency may request an on-demand test through a web interface to a monitoring network. When the RBAM checks in to the monitoring network, the on-demand request is downloaded to the RBAM and the RBAM will power on and prompt the offender to take a breath test.

"Negative Test" means a test where the offender provides a valid sample, and the breath test result or BrAC is less than the LLOD set by the user.

"Positive Test" means a test where the offender provides a valid sample, and the BrAC result of that sample is greater than or equal to the LLOD set by the user. Positive tests can be caused by either actual deep-lung breath alcohol (and therefore blood alcohol), or by mouth alcohol. Therefore, in one embodiment, a confirmation test may be required for all initial positive tests.

"Grace Period" means the time allowed from when the breath test is supposed to be performed to when the offender must start blowing. E.g., if the grace period is ten minutes, an offender can start blowing for a 10:00 AM test as late as 10:10 AM. This is a user selectable variable that may be pulled down from a monitor network by the RBAM or programmed into the RBAM. In one embodiment the proposed range is from two to sixty minutes in increments of one minute, with a default of fifteen minutes. Note that the grace period does not apply to confirmation tests/retests.

"Wait-To-Retest Period" means the time after an initial test that the offender must wait to provide a confirmation test. This is a user selectable variable that may be pulled down from a monitor network by the RBAM or programmed into the RBAM. The wait-to-retest period can be no shorter than the recovery period (see definition below). In one embodiment the proposed range is from two to twenty minutes in increments of one minute, with a default of two minutes.

"Recovery Period" means the minimum time after a valid sample that the offender must wait before providing another breath sample and is hardware driven. E.g., if the fuel cell, and sample system require 83 seconds to "recover," the recovery period must be greater than 83 seconds and could be rounded to 90 or 120 seconds. The recovery period defines the minimum allowable wait-to-retest period.

"Initial Testing Window" means how long the offender has after the RBAM prompts the offender to "BLOW' to provide a valid sample, including all retries. When the initial testing window expires the RBAM locks until the next scheduled test or on-demand test. In one embodiment, the initial testing window is set for five minutes. The initial testing window does not have to be time-based, but could also be determined by limiting the number of retry attempts, or by some combination of time and retry attempts. In another embodiment, the RBAM may be allowed to stay powered up and not locked to allow for a breath test outside of the initial testing window.

"Confirmation Testing Window" is analogous to the initial testing window, but applies to confirmation tests/retests. After the wait-to-retest period has expired, the RBAM will display "Blow" for the confirmation test, and the offender has to provide a valid sample before the confirmation test window expires, including all retries. If the offender does not, then a missed or incomplete confirmation test will be reported. In one embodiment, the confirmation testing window is set for five minutes. The confirmation testing window does not have to be time-based, but could also be determined by limiting the number of retry attempts, or by some combination of time and retry attempts. In another embodiment, the RBAM may be allowed to stay powered up and not locked to allow for a breath test outside of the confirmation testing window.

The testing windows above in one embodiment are configurable variables, but not user selectable. They may be pulled down from a monitor network by the RBAM as part of a configuration file, enabling the option to change them system-wide if needed. For example, if some field experience indicates that the confirmation test window is too long or short, one change can be made to the configuration file and this new change will make its way down to each RBAM over time.

"Circumvention Detected" means any test in which the person blowing into the RBAM is identified not to be the offender, or in which the offender is attempting to provide the breath sample from a source other than the offender's own breath. In both situations, in one embodiment, this determination is made through inspection of the test image(s) or comparison of the test image(s) to enrollment image(s), either manually or via an automated method. In one embodiment, prior to being confirmed as an attempted circumvention detected, test results are labeled as Pending Review. According to a first aspect of the present invention therefore, there is provided a remote breath alcohol monitor configured for capturing a facial image of the user while sampling the user's breath, the monitor comprising:
a vent opening in the remote breath alcohol monitor disposed for receiving a breath sample in an ethanol sensor within the remote breath alcohol monitor;
at least one circuit board assembly within the remote breath alcohol monitor arranged for processing the output of the ethanol sensor to determine a breath alcohol content;
a camera connectable to the at least one circuit board assembly arranged for capturing a facial image of a user;
a wireless cellular phone module embedded in the at least one circuit board assembly and arranged for sending the breath alcohol content and the facial image;
the at least one circuit board assembly being arranged to perform a facial match on the facial image compared to an enrollment image;
a breath tube connectable to the vent;
and characterized by:
   a pressure transducer within a fuel cell assembly with tubing 10 arranged to measure that the offender has blown hard enough and long enough for the breath sample to be valid;
   in that the breath tube is flat in cross-section thus to prevent the insertion of a smaller round tube thereinto and to prevent a user from tilting his/her head too much from side to side, that is no more than about 10°;
   and in that the length of the breath tube is such as to achieve a focal length between the camera and the user's face

According to a second aspect of the invention a method for remote breath alcohol monitoring, the method comprising the steps of:
(a) receiving a first breath sample in the remote breath alcohol monitor of the first aspect of the invention;
(b) analyzing by the remote breath alcohol monitor the first breath sample for a first breath alcohol content;
(c) capturing with a camera in the remote breath alcohol monitor a first facial image;
(d) performing a first facial match on the first facial image compared to an enrollment image; and
(e) sending by the remote breath alcohol monitor the first breath alcohol content and the first facial image to a monitor network, the remote breath alcohol monitor being as per the first aspect of the invention

### BRIEF DESCRIPTION OF THE SEVERAL VIEWS OF THE DRAWINGS

**FIG. 1** shows a chart of the alcohol monitoring continuum ranging from low risk/low cost to high risk/high cost.
**FIG. 2** shows a schematic/block diagram of an overall system in an embodiment of the remote breath alcohol monitor.
**FIGS. 3A-3D** show a flow chart of a general method from the offender's perspective of an embodiment of remote breath alcohol monitoring.
**FIGS. 4A-4B** show exploded views of an embodiment of a remote breath alcohol monitor.
**FIGS. 5A-5C** show various assembled views of an embodiment of a remote breath alcohol monitor.
**FIG. 6** shows a screen capture from the display in an embodiment of a remote breath alcohol monitor.
**FIGS. 7A-7D** show a flow chart of a general method of implementation in an embodiment of remote breath alcohol monitoring.
**FIG. 8** shows a block diagram of the circuit boards in an embodiment of a remote breath alcohol monitor.
To assist in the understanding of the present disclosure the following list of components and associated numbering found in the drawings is provided herein:

**Table of Components**

| **Component** | **#** |
|---|---|
| Housing Front Panel | **1** |
| Housing Back Panel | **2** |
| Housing Battery Door | **3** |
| Window Assembly | **4** |
| Switch/Indicator Panel | **5** |
| Battery Pack | **6** |
| Main Circuit Board Assembly | **7** |
| Processor Circuit Board Assembly | **8** |
| Camera Circuit Board Assembly | **9** |
| Fuel Cell Assembly With Tubing | **10** |
| OLED Display | **11** |
| Pump | **12** |
| RF Shield | **13** |
| Breath Tube | **14** |
| USB Connector | **15** |
| Charging Connection | **16** |
| Mirrors | **17** |
| Camera | **18** |
| Mute Switch | **19** |
| Visual Speaker Indicator | **20** |
| Power On/Off Switch | **21** |
| Battery Indicator | **22** |
| Visual Battery Indicator | **23** |
| Speaker | **24** |
| Vent Opening | **25** |
| Connectors | **26** |
| Remote Breath Alcohol Monitor (RBAM) | **200** |
| Offender | **202** |
| Cellular Network | **204** |
| Monitor Network | **206** |
| Monitoring Station | **208** |
| Supervising Agency | **210** |
| Wireless Communication Link | **212** |
| Communication Link | **214** |
| Internet | **216** |
| Communication Link | **218** |
| Internet Connection | **220** |
| Communication Link | **222** |
| Method | **300** |
| Initial Test Routine | **315** |
| Initial Retry Test Subroutine | **329** |
| Confirmation Test Routine | **353** |
| Confirmation Retry Test Subroutine | **367** |
| Messages | **602** |
| Time of Day | **604** |
| Cellular Signal Strength | **606** |
| Battery Strength | **608** |

### DETAILED DESCRIPTION

Referring now to the figures, like reference numerals and names refer to structurally and/or functionally similar elements thereof, and if objects depicted in the figures that are covered by another object, as well as the tag line for the element number thereto, may be shown in dashed lines. **Figure 1** shows a chart of the alcohol monitoring continuum ranging from low behavioral risk/low monitoring cost to high behavioral risk/high monitoring cost. Referring now to **FIG. 1** and moving from left to right, low risk offenders may be required to have an ignition interlock device or breath alcohol ignition interlock device (IID and BAIID) installed on their vehicle. An IID or BAIID is a mechanism, like a breathalyzer, installed on a motor vehicle's dashboard. Before the vehicle's engine can be started, the driver first must exhale into the device. If the resultant BrAC is greater than the programmed BrAC (which varies between jurisdictions), the device prevents the engine from being started.

Next on the continuum is a random testing program. Typical programs require the offender to submit to random alcohol and drug screens on the days they appear for their status hearings and random days between court appearances and in some cases unannounced home visits by a probation officer.

A more rigorous testing program on the alcohol monitoring continuum has an abstinence requirement and requires an offender to report to a local sheriff's office or other designated location twice daily for alcohol testing. Typical times are between 7 a.m. and 9 a.m. and between 7 p.m. and 9 p.m.

Next on the continuum is the use of alcohol biomarkers, which are physiological indicators of alcohol exposure or ingestion. Alcohol biomarkers are generally divided into indirect and direct biomarkers. Indirect alcohol biomarkers suggest heavy alcohol use by detecting the toxic effects that alcohol may have had on organ systems or body chemistry. Direct alcohol biomarkers are analytes of alcohol metabolism. Direct alcohol biomarkers include alcohol itself and ethyl glucuronide (EtG).

Continuous alcohol monitoring (CAM) is next on the alcohol monitoring continuum scale. CAM is accomplished with a transdermal alcohol monitoring device that is worn on the body of the offender. One such device is the Secure Continuous Remote Alcohol Monitor, or SCRAM^{®}, from Alcohol Monitoring Systems, Inc., Littleton, Colorado. Transdermal alcohol monitoring means that alcohol is measured "through the skin," or by the content of alcohol present in the insensible perspiration that is constantly given off by the skin. If an offender has been drinking, it shows up in the level of ethanol vapor present in this insensible perspiration.

Next on the continuum is the use of CAM combined with house arrest. House arrest (also called home arrest, home confinement, home detention, curfew monitoring, or electronic monitoring) is a measure by which a person is confined by the authorities to a certain residence. Travel is usually restricted, if allowed at all. House arrest is a lenient alternative to prison time. House arrest is often enforced through the use of technology devices or services. One method is an electronic sensor locked to the offender's ankle (technically called an ankle monitor, and sometimes referred to as a tether). Typically, the electronic sensor transmits a radio frequency signal to a fixed base station. The base station is connected to a police facility or a monitoring service. Most programs allow employed offenders to continue to work, and only confine them to their house during non-working hours. House arrest can also be accomplished with GPS tracking bracelets. Some devices combine CAM with house arrest tracking in a single device, such as SCRAM^{®} from Alcohol Monitoring Systems, Inc., Littleton, Colorado. Other devices exist that combine CAM with GPS tracking.

Next, pharma-injectible drugs may be used for some high behavioral risk offenders. After being injected, if the offender drinks alcohol, these drugs produce a physical reaction that may include flushing, nausea, vomiting, and headaches.

Finally on the alcohol monitoring continuum scale, jail or prison may be the only option for the highest behavioral risk offenders. This option is also the most expensive.

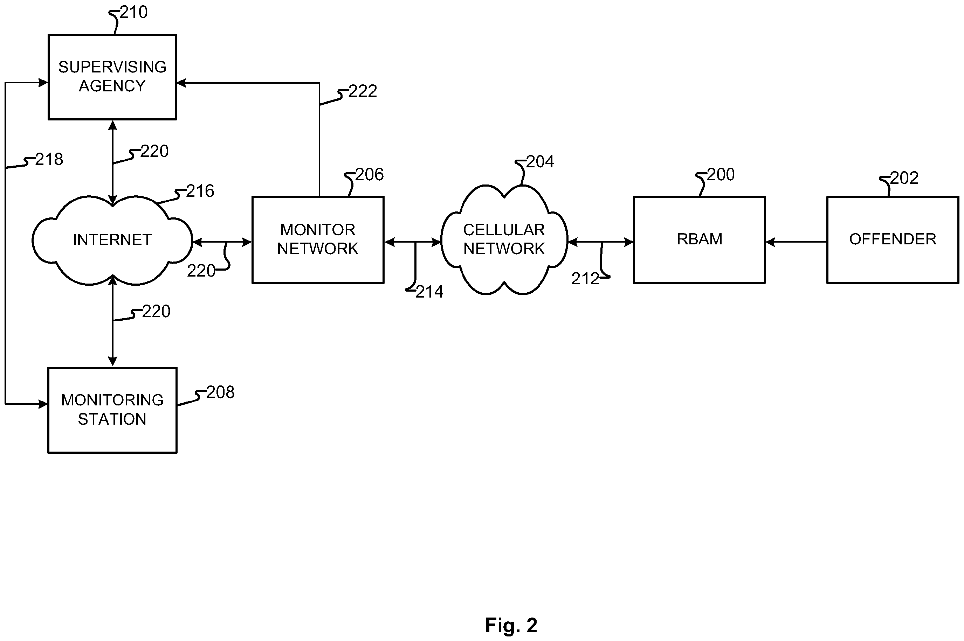

The use of RBAM **200** (*see* **FIG. 2**) that is the subject of this disclosure is positioned toward the low behavior risk and low monitoring cost on the alcohol monitoring continuum. The typical candidate for RBAM **200** would be a first time or second time DUI offender or one who has committed a public order offense. The offender would typically have a low blood alcohol content (BAC) or BrAC at the time of arrest. For those offenders who start out on a higher risk/higher cost alternative, their compliance with those standards would allow them to earn the right to this lower cost and less intrusive alcohol monitoring solution.

**Figure 2** shows a schematic/block diagram of the overall system of an embodiment of remote breath alcohol monitoring. Referring now to **FIG. 2**, in one embodiment RBAM **200** is a portable handheld wireless breath alcohol tester with built in cellular, GPS, and facial recognition capabilities that is designed to be rugged for everyday use in the corrections environment. RBAM **200** is used by the Offender **202** being monitored in a manner to be described below. RBAM **200** can be carried around by Offender **202** throughout the day and night, just like a typical cell phone. There are a number of anti-tamper features designed into RBAM **200** to ensure that the breath tests taken are from Offender **202**, and accurately represent the breath alcohol level of Offender **202** and not some other person. Though this discussion focuses on one Offender **202**, one skilled in the art will recognize that many RBAM **200**s may be used by many Offender **202**s at the same time over a broad geographic area, and all may be monitored by Monitor Network **206**, which is the intended purpose. Likewise, there may be multiple Monitor Networks **206** and Monitoring Stations **208** that manage additional Offender **202s** in diverse geographic locations.

Offender **202** will be instructed by RBAM **200** to provide breath tests that are time stamped at scheduled or random intervals during any given twenty-four hour period, and could be seven days a week, 365 days a year, or any given set of days. There may be days when no tests are required. Testing schedules may vary from one Offender **202** to another Offender **202**. Offender **202** typically knows when the request to give fixed breath tests will occur. Random monitoring eliminates the ability for Offender **202** to manipulate drinking patterns to avoid detection. Offender **202** typically does not know when the request to take random breath tests will occur.

Breath tests taken as scheduled, or randomly, are uploaded at the conclusion of each breath test along with the location fix, facial match results, and an image of Offender **202** taken while blowing. All of these are collectively referred to as test results. RBAM **200** places a call via Wireless Communication Link **212** to Cellular Network **204**. Cellular Network **204** completes the call via Communication Link **214** to Monitor Network **206**. In practice, Cellular Network **204** may actually be many different networks, including the Internet, interoperating with each other but is shown as a single network for simplicity. Communication Link **214** may be a wireless link or a combination of a wireless and a wired link. If there is no Wireless Communication Link **212** at the location where the breath test was taken, RBAM **200** will store the test results and send the test results when a Wireless Communication Link **212** becomes available. Once a Wireless Communication Link **212** is established, RBAM **200** calls Monitor Network **206** via Cellular Network **204** and Communication Link **214**. Monitor Network **206** validates the identity of RBAM **200** and authenticates the test results before it is permanently stored. Monitor Network **206** then analyzes the test results received and separates and groups the test results into a number of separate categories for reporting to monitoring personnel at Monitoring Station **208**. The test results can then be accessed by the monitoring personnel through the use of secured dedicated websites through the Internet **216** and Internet Connection **220** to Monitor Network **206**. When Monitor Network **206** analyzes the test results received, an automatic alert, based upon predetermined, stored rules, may be sent directly from Monitor Network **206** to a call center at Supervising Agency **210** over Communication Link **222**, or to an individual previously designated by Supervising Agency **210**, when a specific alert, or combination of alerts, are received. The alert may be an e-mail, text message, or a page to a previously provided number. Communication Link **222** may be a wire or wireless connection. The term "server" used with respect to Monitor Network **206** may be one hardware device partitioned into many functional virtual servers, such as a central server, database server, rules-based server, etc., or it may be several hardware devices dedicated to a particular function, each in communication with each other.

Monitoring Station **208** may be located at Monitor Network **206**, or in a separate location as shown in **FIG. 2**. Monitoring personnel at Monitoring Station **208** have access to all of the data gathered on all of the Offenders **202**. Supervising personnel at the call center of Supervising Agency **210**, however, only have access to those Offender **202**s that are associated with Supervising Agency **210**.

Monitoring Station **208** may automatically or periodically transmit data received from RBAM **200** via Cellular Network **204** to Monitor Network **206** to one or more persons at Supervising Agency **210** who are assigned to monitor Offender **202**, such as a parole officer, probation officer, case worker, or other designated person or persons in charge of enrolling Offender **202** and monitoring the data being collected on Offender **202**. Only one Supervising Agency **210** is shown for simplicity, but one skilled in the art will recognize that many Supervising Agencies **210** may be accessing Monitor Network **206** at any given time. A connection is established with Supervising Agency **210** through Communication Link **218**. Typically this connection is accomplished via the telephone system through a wire or wireless link, and may connect to a pager or cellular phone of the designated person or via email. Designated personnel at Supervising Agency **210** may also access Monitor Network **206** through the use of secured dedicated websites through the Internet **216** and Internet Connection **220** to Monitor Network **206**. Monitor Network **206** hosts a website that allows Supervising Agency **210** the ability to log on and track Offender **202** compliance in a manner most suitable to the needs of Supervising Agency **210**, and can be defined to fit the needs of both small and large programs. Each Supervising Agency **210** may customize the frequency of testing and the method of notification for alerts that they want to receive from Monitor Network **206**. Alerts may be categorized by the type and severity of alert, allowing each Supervising Agency **210** to prioritize and better categorize a response (*e*.*g*., a low battery warning versus a possible alcohol consumption violation).

Each Supervising Agency **210** has its own separate data storage area on the database server at Monitor Network **206** so that representatives from each Supervising Agency **210** can retrieve the secure data they need when they need it.

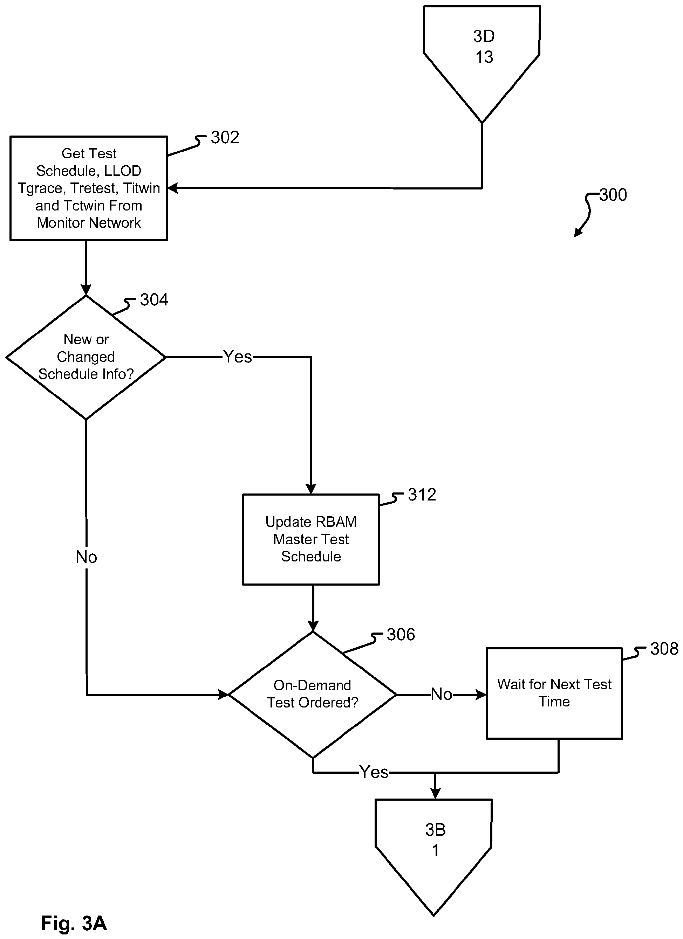

**Figures 3A-3D** show a flow chart of a general method from the offender's perspective of an embodiment of remote breath alcohol monitoring. Referring now to **FIG. 3A**, Method **300** begins in block **302** where an RBAM **200** assigned to a particular Offender **202** obtains the testing schedule, the Lower Limit of Detection (LLOD), the grace period designated as T_{grace}, the wait-to-retest period designated as Tᵣₑₜₑₛₜ, the initial testing window designated as T_{itwin}, and the confirmation testing window designated as T_{ctwin}, for this particular Offender **202** from Monitor Network **206**. Decision block **304** determines if there is a new testing schedule or a changed testing schedule from what was previously stored in RBAM **200**. If no, then decision block **306** determines if an on-demand test has been ordered. If no, then RBAM **200** in block **308** waits for the next scheduled test time from the RBAM master test schedule to arrive.

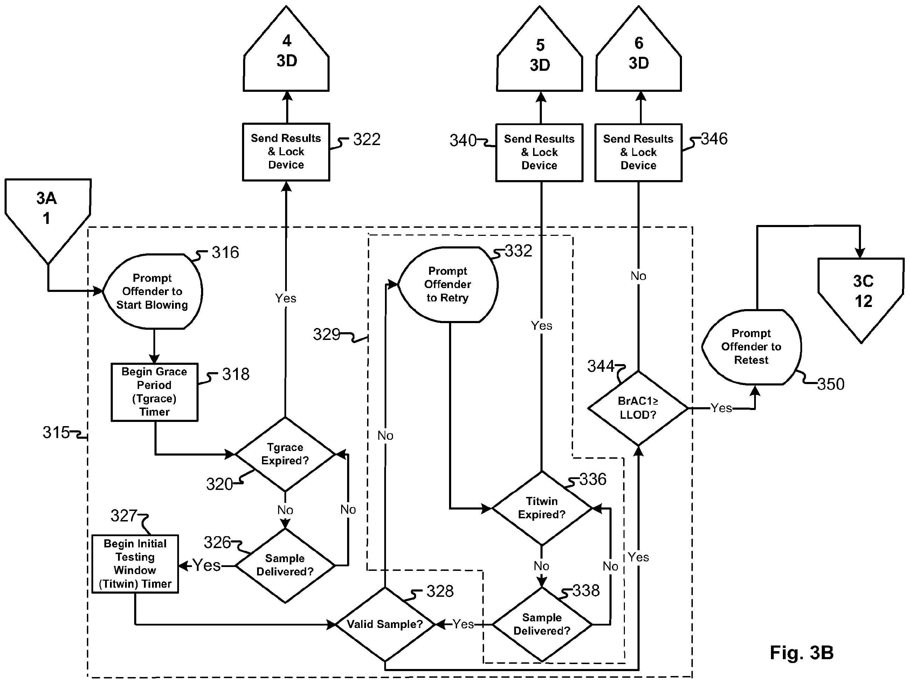

If the answer in decision block **304** is yes, then in block **312** the RBAM master test schedule in RBAM **200** is updated and stored in RBAM **200**. If the answer in decision block **306** is yes, or, the next scheduled test time has arrived in block **308**, control passes to block **316** in **FIG. 3B**.

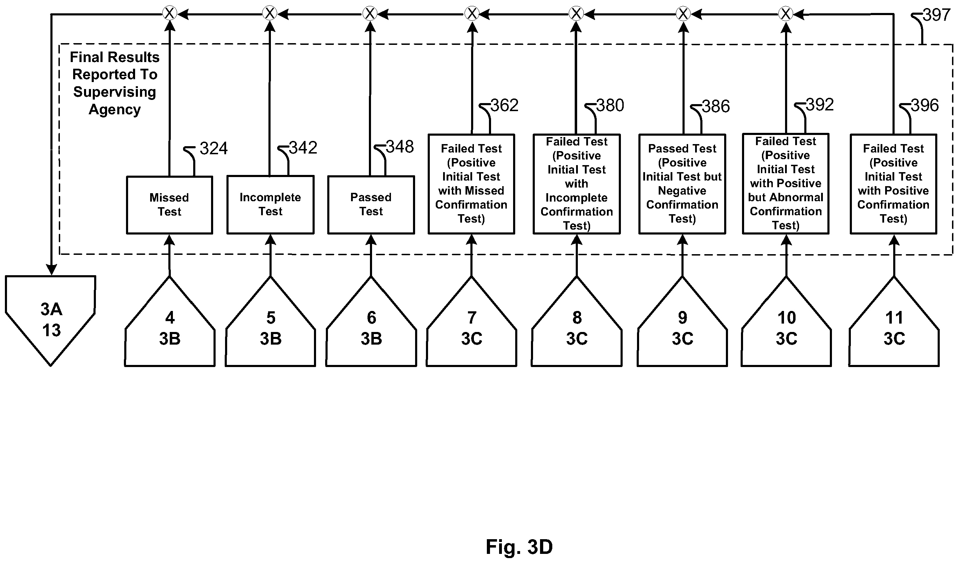

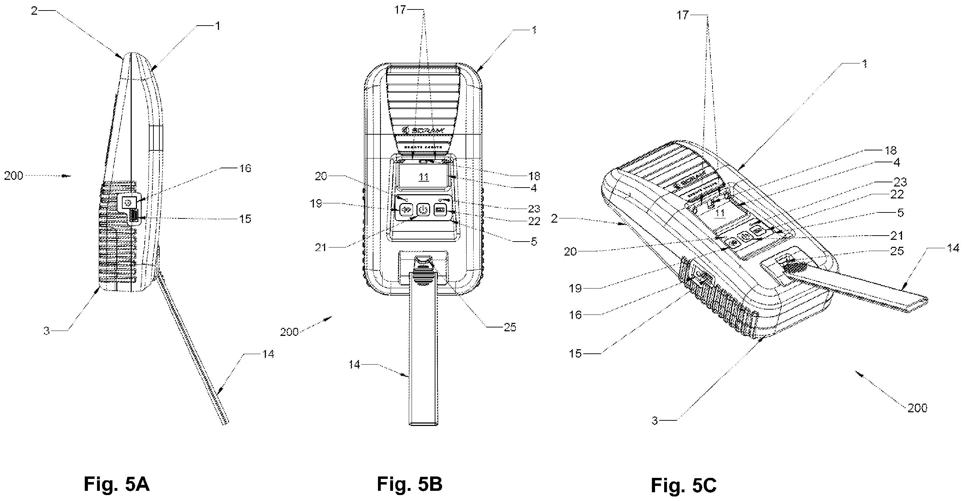

Referring now to **FIG. 3B**, the steps enclosed within Dashed Line Box **315** encompass the Initial Test Routine. In block **316** a message is displayed to Offender **202** on OLED Display **11** (see **FIGS. 5B** and **5C**) such as "BLOW' which indicates that Offender **202** should insert Breath Tube **14** into RBAM **200** and begin blowing into Breath Tube **14**. In block **318** the timer for the grace period (T_{grace}) is started. Decision block **320** determines if T_{grace} has expired. If yes, this test result is sent to Monitor Network **206** in block **322** and RBAM **200** is locked, and control passes to block **324** in **FIG. 3D****.**

Referring now to **FIG. 3D**, in block **324** Monitor Network **206** sends a message to Supervising Agency **210** that a test was missed by Offender **202** if missed tests have been selected for immediate notification. The test results are stored in a server in Monitor Network **206**. Control then returns to block **302** in **FIG. 3A**.

Referring back now to **FIG. 3B**, if decision block **320** determines that T_{grace} has not expired, then decision block **326** determines if a breath sample has been delivered by Offender **202** into RBAM **200**. If no, then control returns to decision block **320** to determine if T_{grace} has expired. If decision block **326** determines that a breath sample has been delivered by Offender **202** into RBAM **200**, then in block **327** the timer for the initial testing window (T_{itwin}) is started. Decision block **328** determines if the breath sample is a valid sample. If no, then control passes to the steps within Dashed Line Box **329** encompassing the Retry Subroutine. In block **332** a message is displayed to Offender **202** on OLED Display **11** asking Offender **202** to retry taking a breath test. Decision block **336** determines if T_{itwin} has expired. If no, decision block **338** determines if a breath sample has been delivered. If yes, control returns to decision block **328** to determine if the breath sample is valid. If decision block **338** determines that no breath sample has been delivered, control returns to decision block **336** which determines if T_{itwin} has expired. If no, control returns to decision block **338** to determine if a breath sample has been delivered. If T_{itwin} has expired in decision block **336**, this test result is sent to Monitor Network **206** in block **340** and RBAM **200** is locked, and control passes to block **342** in **FIG. 3D**.

Referring now to **FIG. 3D**, in block **342** Monitor Network **206** sends a message to Supervising Agency **210** that an incomplete test has occurred for Offender **202** if incomplete tests have been selected for immediate notification. The test results are stored in a server in Monitor Network **206**. Control then returns to block **302** in **FIG. 3A**.

Referring back now to **FIG. 3B**, if decision block **328** determines that a valid sample has been received, then decision block **344** determines if the **BrAC1** (the breath alcohol content from the initial breath test) is greater than or equal to the LLOD. If no, this test result is sent to Monitor Network **206** in block **346** and RBAM **200** is locked, and control passes to block **348** in **FIG. 3D**.

Referring now to **FIG. 3D**, in block **348** Monitor Network **206** sends a message to Supervising Agency **210** that a passed test has occurred for Offender **202** if passed tests have been selected for immediate notification (typically not the case). However, if the facial match is negative for this test, the test is labeled as Pending Review, and Monitor Network **206** sends a message to Supervising Agency **210** that a failed test with circumvention detected has occurred if this test result has been selected for immediate notification. The test results are stored in a server in Monitor Network **206.** Control then returns to block **302** in **FIG. 3A**.

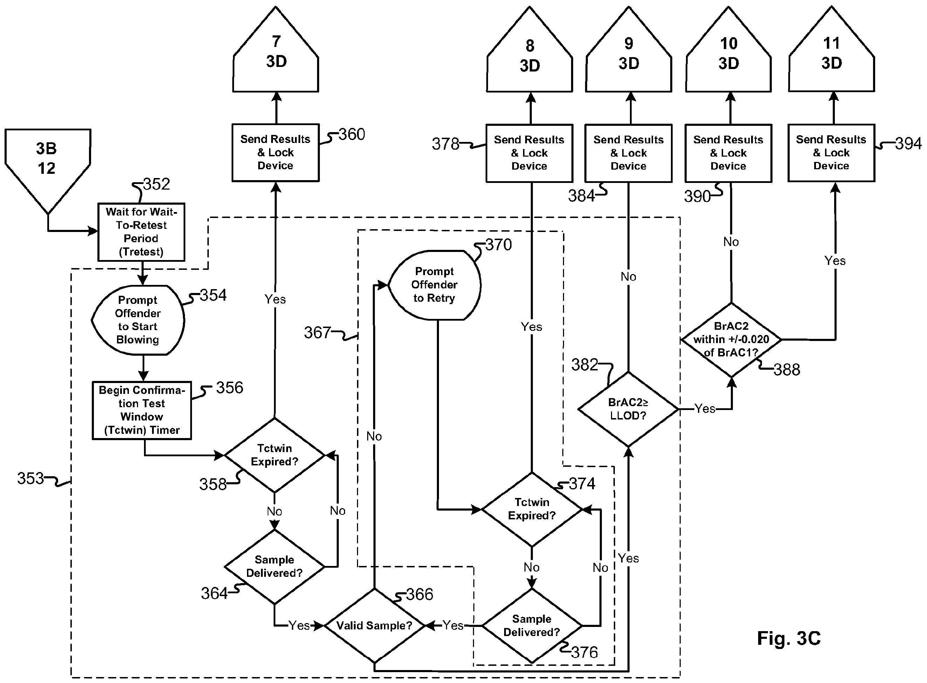

Referring back now to **FIG. 3B**, if decision block **344** determines that the **BrAC1** is greater than or equal to the LLOD, indicating a failed breath test, then in block **350** a message is displayed to Offender **202** on OLED Display **11** asking Offender **202** to retest. Control then passes to block **352** in **FIG. 3C**.

Referring now to **FIG. 3C**, the steps enclosed within Dashed Line Box **353** encompass the Confirmation Test Routine. In block **352** the timer for the wait-to-retest period (Tᵣₑₜₑₛₜ) is started. Once Tᵣₑₜₑₛₜ has passed, in block **354** a message is displayed to Offender **202** on OLED Display **11** asking Offender **202** to start blowing. In block **356** the timer for the confirmation testing window (T_{ctwin}) is started. Decision block **358** determines if T_{ctwin} has expired. If yes, the test result is sent to Monitor Network **206** in block **360** and RBAM **200** is locked, and control passes to block **362** in **FIG. 3D**.

Referring now to **FIG. 3D**, in block **362** Monitor Network **206** sends a failed test message to Supervising Agency **210**, which in this case was a positive initial test (indicating alcohol) that was followed by a missed confirmation test for Offender **202** if failed tests have been selected for immediate notification. However, if the facial match is negative for the initial test, the test event is labeled as Pending Review, and Monitor Network **206** sends a message to Supervising Agency **210** that a failed test with circumvention detected has occurred if this test result has been selected for immediate notification. The test results are stored in a database server in Monitor Network 206. Control then returns to block **302** in **FIG. 3A**.

Referring back now to **FIG. 3C**, if decision block **358** determines that T_{ctwin} has not expired, then decision block **364** determines if a valid breath sample has been delivered by Offender **202** into RBAM **200**. If no, then control returns to decision block **358** to determine if T_{ctwin} has expired. If in decision block **364** a breath sample has been delivered, decision block **366** determines if the breath sample is valid. If no, then control passes to the steps within Dashed Line Box **367** encompassing the Confirmation Retry Test Subroutine. In block **370** a message is displayed to Offender **202** on OLED Display **11** asking Offender **202** to retry taking a breath test. Decision block **374** determines if T_{ctwin} has expired. If no, decision block **376** determines if a breath sample has been delivered. If yes, control returns to decision block **366** to determine if the breath sample is valid. If decision block **376** determines that no breath sample has been delivered, control returns to decision block **374** which determines if T_{ctwin} has expired. If yes, the result is sent to Monitor Network **206** in block **378** and RBAM **200** is locked, and control passes to block **380** in **FIG. 3D**.

Referring now to **FIG. 3D**, in block **380** Monitor Network **206** sends a failed test message to Supervising Agency **210**, which in this case was a positive initial test (indicating alcohol) that was followed by an incomplete confirmation test for Offender **202** if failed tests have been selected for immediate notification. However, if the facial match is negative for the initial test, the test is labeled as Pending Review, and Monitor Network **206** sends a message to Supervising Agency **210** that a failed test with circumvention detected has occurred if this test result has been selected for immediate notification. The test results are stored in a database server in Monitor Network **206**. Control then returns to block **302** in **FIG. 3A**.

Referring back now to **FIG. 3C**, if decision block **366** determines that the breath sample is valid, decision block **382** determines if the **BrAC2** (the breath alcohol content from the confirmation breath test) is greater than or equal to the LLOD. If no, this result is sent to Monitor Network **206** in block **384** and RBAM **200** is locked, and control passes to block **386** in **FIG. 3D**.

Referring now to **FIG. 3D**, in block **386** Monitor Network **206** sends a passed test message to Supervising Agency **210**, which in this case was a positive initial test (indicating alcohol) that was followed by a negative confirmation test for Offender **202** if passed tests have been selected for immediate notification (typically not the case). However, if the facial match is negative for the initial test or the retest, the test is labeled as Pending Review, and Monitor Network **206** sends a message to Supervising Agency **210** that a failed test with circumvention detected has occurred if this test result has been selected for immediate notification. The test results are stored in a database server in Monitor Network **206**. Control then returns to block **302** in **FIG. 3A**.

Referring back now to **FIG. 3C**, if decision block **382** determines that the **BrAC2** is greater than or equal to the LLOD, then decision block **388** determines if the **BrAC2** is, in one embodiment, within plus or minus 0.020% of **BrAC1**. This value, which is a system variable not changeable by Supervising Agency **210**, determines an abnormal test. When the BrAC difference between the initial test and the confirmation test is greater than 0.02%, there is most likely mouth alcohol in one or both of the tests, or it could be a different person taking one of the tests. The human body in the short period of time between the initial test and the confirmation test cannot burn off that much alcohol. The value used for abnormal tests could be any other value or predetermined criteria based on particular legal, policy, or supervisory needs. If no, then this result is sent to Monitor Network **206** in block **390** and RBAM **200** is locked, and control passes to block **392** in **FIG. 3D**.

Referring now to **FIG. 3D**, in block **392** Monitor Network **206** sends a failed test message to Supervising Agency **210**, which in this case was a positive initial test (indicating alcohol) that was followed by a positive (indicating alcohol) but an abnormal confirmation test for Offender **202** if failed tests have been selected for immediate notification. However, if the facial match is negative for the initial test or the retest, the test event is labeled as Pending Review, and Monitor Network **206** sends a message to Supervising Agency **210** that a failed test with circumvention detected has occurred if this test result has been selected for immediate notification. The test results are stored in a database server in Monitor Network **206**. Control then returns to block **302** in **FIG. 3A**.

Referring back now to **FIG. 3C**, if decision block **388** determines that the **BrAC2** is, in one embodiment, within plus or minus 0.020% of **BrAC1**. This value, which is a system variable not changeable by Supervising Agency **210**, could be any other value or predetermined criteria based on particular legal, policy, or supervisory needs. This result is sent to Monitor Network **206** in block **394** and RBAM **200** is locked, and control passes to block **396** in **FIG. 3D**.

Referring now to **FIG. 3D**, in block **396** Monitor Network **206** sends a failed test message to Supervising Agency **210**, which in this case was a positive initial test (indicating alcohol) that was followed by a positive confirmation test for Offender **202**. However, if the facial match is negative for the initial test or the retest, the test is labeled as Pending Review, and Monitor Network **206** sends a message to Supervising Agency **210** that a failed test with circumvention detected has occurred if this test result has been selected for immediate notification. The test results are stored in a database server in Monitor Network **206**. Control then returns to block **302** in **FIG. 3A**.

The steps enclosed within Dashed Line Box **397** encompass all of the different final test results received from RBAM **200** that may be sent to Supervising Agency 210 in one embodiment, subject to modification by the results of the facial match. Other embodiments may have different final test results and use facial match results differently.

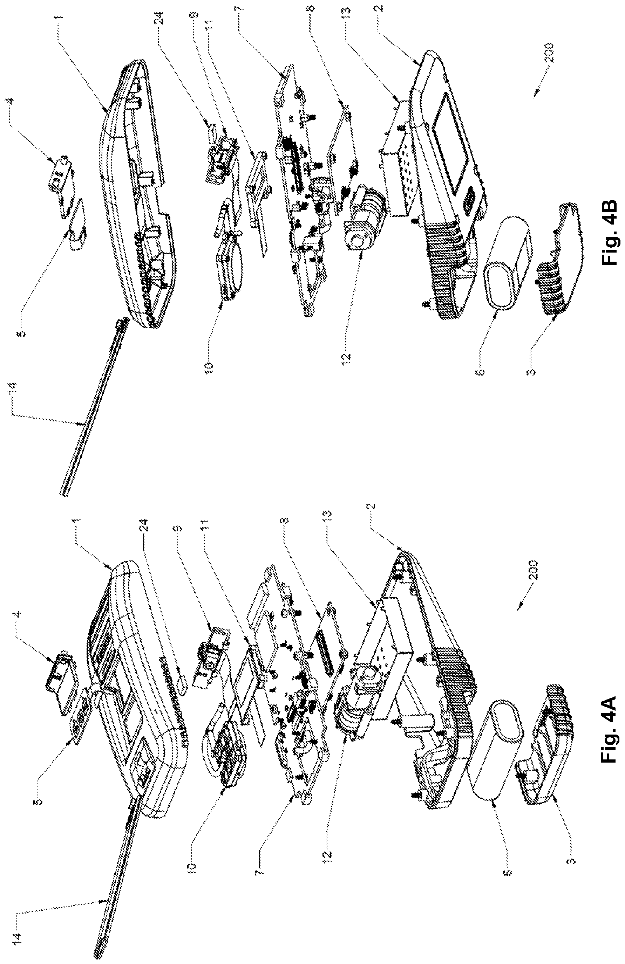

**Figures 4A** and **4B** show exploded views of an embodiment of a remote breath alcohol monitor. Referring now to **FIGS. 4A** and **4B**, RBAM **200** has a Housing Front Panel **1** and a Housing Back Panel **2**. Battery Pack **6** fits within Housing Back Panel **2** and is secured within Housing Back Panel **2** by Housing Battery Door **3**. Battery Pack **6** provides power for all the functions of RBAM **200**. Battery Pack **6** in one embodiment is a rechargeable battery. Battery Pack **6** is not replaceable by Offender **202** but can be replaced by Supervising Agency **210** or other authorized personnel.

Window Assembly **4** and the Switch/Indicator Panel **5** fit within a midway opening in Housing Front Panel **1**. Window Assembly **4** and Switch/Indicator Panel **5** are shown in more detail in **FIG. 5** and are discussed in detail below.

Offender **202** is instructed to take a breath test through output displayed on OLED Display **11**. In other embodiments other types of displays, sound tones, or tactile feedback, may be used to instruct Offender **202** to take a breath test. Offender **202** then inserts Breath Tube **14** into an opening in a lower portion of Housing Front Panel **1**. Breath Tube **14** was designed to be flat instead of round as is typical in prior art breathalyzers on the market today. The flat design of Breath Tube **14** prevents offenders from circumventing the breath test by inserting a smaller round tube inside the larger round breathalyzer tube, and having someone else blow into the smaller round tube into the breathalyzer. There simply is no room in Breath Tube **14** to insert another tube.

When the user is instructed to blow as displayed on OLED Display **11**, the breath from the user travels through Breath Tube **14** and into tubing that is a part of Fuel Cell Assembly With Tubing **10**. Offender **202** must blow hard enough and long enough as measured by a pressure transducer within Fuel Cell Assembly With Tubing **10** for the breath sample to be valid. Once the pressure requirement is met, Pump **12** pumps a portion of the breath sample to the fuel cell within Fuel Cell Assembly With Tubing **10**. The bulk of the breath sample is vented out of RBAM **200** through Vent Opening **25** (see **FIGS. 5B** and **5C**) in the lower portion of Housing Front Panel **1**. The electrical signal registered in the fuel cell, whose signal strength is proportional to the alcohol content of the breath sample, is sent to Main Circuit Board Assembly **7**. Main Circuit Board Assembly **7** in one embodiment has a low power processor from the MSP family of processors available from Texas Instruments and runs continuously, allowing Main Circuit Board Assembly **7** to perform processes in the background without waking up RBAM **200**. Other embodiments could use a different processor.

The digital image taken by Camera **18** is sent to Processor Circuit Board Assembly **8**. In one embodiment, Processor Circuit Board Assembly **8** has an i.MX family of processors available from Freescale Semiconductor and runs the Android operating system. Other embodiments could use a different processor, different operating system, or no operating system. Processor Circuit Board Assembly **8** runs a camera module, and a wireless cellular phone module embedded in Main Circuit Board Assembly **7**. In one embodiment, the wireless cellular phone module is a Cinterion 3G GSM module. One skilled in the art will recognize that a CDMA module could also be used, as well as other wireless communication technologies including Wi-Fi, Bluetooth, ZigBee, and others. In one embodiment, the Processor Circuit Board Assembly processes the image from the camera to perform facial matching. Thus the software for performing facial matching is run on Processor Circuit Board Assembly **8** in RBAM **200** as opposed to sending the image over a communications network to a central server for facial match processing. In another embodiment, the facial matching software is stored on a server at Monitoring Network **206** and the image may be uploaded to Monitoring Station **208** to perform facial matching. In another embodiment, part of the facial matching process is performed on Processor Circuit Board Assembly **8** in RBAM **200** and part by Monitoring Network **206**. In another embodiment, facial matching may be performed by a third party service provider. That is, data required for facial matching is communicated to the third party service provider, and the results of the facial match are communicated by the third party service provider back to RBAM **200** or Monitoring Network **206** or both. The image uploaded may be at a lower resolution than what the facial matching software uses to perform a facial match. The lower resolution image saves on uploading costs but still gives Supervising Agency **210** personnel the ability to compare the uploaded image with an enrollment image to verify that the right person took the breath test.

Liveness face detection can prevent breaching the system with printed photographs of Offender **202** placed in front of RBAM **200**. Liveness face detection takes a video, or several different still images, to detect eye movement, mouth movement, etc., to determine that a real face is being viewed prior to taking the image when Offender **202** is blowing into Breath Tube **14**. Due to its high power consumption, Processor Circuit Board Assembly **8** is only powered on when needed, such as when a breath sample is being taken, an image is taken and processed for facial recognition, and the results are sent via the wireless cellular phone module to Monitor Network **206**. Through GPS technology in the wireless cellular phone module, or through backup location technologies available from the cellular carrier's network, the location fix of Offender **202** is also transmitted to Monitor Network **206**. Power management in RBAM **200** is unique, using a low power processor to perform some functions coupled with a high power processor to perform other functions, in order to conserve battery power. Other embodiments could use just a single processor instead of the two processors described herein. Processor Circuit Board Assembly **8** is protected from radio frequency electromagnetic radiation by RF Shield **13**.

**Figures 5A-5C** show various assembled views of an embodiment of a remote breath alcohol monitor. Referring now to **FIGS. 5A-5C**, USB Connector **15** allows RBAM **200** to be connected to a PC so that a variety of functions can be performed, such as firmware upgrades, diagnostic testing, troubleshooting, calibration, etc. The PC accesses Monitor Network **206** via Internet **216** to download firmware updates and onto RBAM **200** through USB Connector **15**. Charging Connection **16** allows RBAM **200** to be plugged in to a charger (not shown) to recharge Battery Pack **6**.

Mirrors **17** in Window Assembly **4** help Offender **202** to properly align Offender **202**'s face so that an image can be taken with Camera **18** mounted within Camera Circuit Board Assembly **9**. If Offender **202** can see his/her face in the Mirrors **17**, then Offender **202** knows that he/she has achieved correct alignment with RBAM **200**. The lens of Camera **18** seats within an opening in Window Assembly **4** between Mirrors **17**. In one embodiment, Camera **18** is a high resolution camera, capable of taking both still images and video, using CMOS technology. Other embodiments can use other types of cameras such as CCD devices. While Offender **202** is delivering a breath sample, Camera **18** in Camera Circuit Board Assembly **9** takes an image of Offender **202**. One skilled in the art will recognize that it may not be practical to take the image at the precise instant the breath sample is delivered, so this image could be taken at the beginning, middle, end, or any reasonable time period before or after the instant the breath sample is delivered. The Camera Circuit Board Assembly may have light sensors in it to adjust the camera module to the current lighting environment, or it may always use a flash regardless of the current lighting environment to control the exposure. The length of Breath Tube **14** is designed to place the face of Offender **202** at the right focal distance from the lens of Camera **18**, which is approximately eight inches (20.32cm). In addition, the flat design of Breath Tube **14** prevents Offender **202** from tilting her/her head too much from side-to-side. Movement from side-to-side needs to be limited to no more than about ten degrees for facial recognition purposes. OLED Display **11** of Switch/Indicator Panel **5** will output, at various times, various commands, status, and information to Offender **202**.

Switch/Indicator Panel **5** has Mute Switch **19** that Offender **202** can press to mute Speaker **24** at any time. Visual Speaker Indicator **20** turns red when muted. Pressing Mute Switch **19** again will un-mute Speaker **24**.

Switch/Indicator Panel **5** also has Power On/Off Switch **21**. Offender **202** can press and hold Power On/Off Switch **21** for a few seconds to force RBAM **200** to power on and check in with Monitor Network **206**, and then RBAM **200** will turn itself off after downloading any schedule changes or other information.

Switch/Indicator Panel **5** also has Battery Indicator **22**. Visual Battery Indicator **23** turns red when Battery Pack **6** needs charging. When plugged in and charging, Visual Battery Indicator **23** turns yellow, and turns green when fully charged and still plugged in via Charging Connection **16** to the charger. When fully charged and unplugged, no color is displayed by Visual Battery Indicator **23**. RBAM **200** will notify Monitor Network **206** if the remaining life of Battery Pack **6** is estimated to be less than a certain percent of battery capacity. RBAM **200** may also provide tactile feedback (vibration) to Offender **202** at various points of operation.

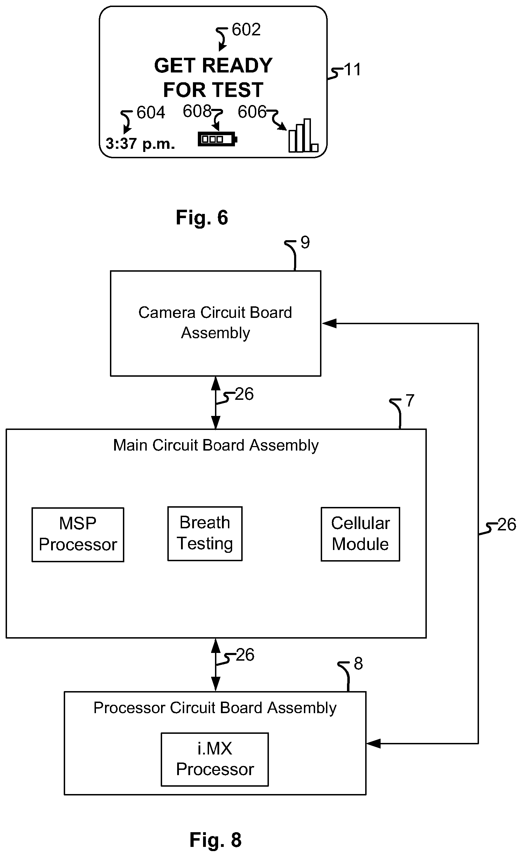

**Figure 8** shows a block diagram of the circuit boards in an embodiment of a remote breath alcohol monitor. Referring now to **FIG. 8**, Main Circuit Board Assembly **7**, Processor Circuit Board Assembly **8**, and Camera Circuit Board Assembly **9** communicate with each other through Connectors **26**. These could all be on one circuit assembly, two circuit assemblies, or any other number as a design choice. The MSP low power processor, the breath testing components, and the cellular phone module are all physically located on Main Circuit Board Assembly **7**. The MSP processor controls the scheduling of breath tests and initiates communication with Monitor Network **206** through the cellular phone module. The i.MX processor is physically located on Processor Circuit Board Assembly **8**. The i.MX processor controls the taking of a breath sample, taking the image, and in one embodiment, performing facial matching, and certain communications with Monitor Network **206**.

**Figure 6** shows a screen capture from the display in an embodiment of a remote breath alcohol monitor. Referring now to **FIG. 6**, when on, OLED Display **11** will display various messages to Offender **202** during a course of operation, such as Message **602**. The Time of Day **604** from the clock within RBAM **200** may also be displayed along with the Cellular Signal Strength **606** which is displayed with one to four vertical bars when OLED Display **11** is on. Battery Strength **608** is displayed with one to four boxes when OLED Display **11** is on.

Described now is just one series of messages. One skilled in the art will recognize that many different messages for many different purposes may be programmed into the operation of RBAM **200**, and this discussion is simply exemplary of one purpose, and the individual messages may be different from that described below.

For a typical breath test, a series of sequential Messages **602** are displayed on OLED Display **11** which are encompassed in the steps shown in blocks **316**, **332**, **350**, **354**, and **370** in **FIGS. 3B** and **3C**. The first Message **602**, "PLEASE WAIT" is displayed for a short period of time before the test. When it is time to begin the test, Message **602** will change to "BLOW' and is displayed on OLED Display **11** (block **316** in **FIG. 3B**). If after a certain period of time, such as two minutes, if Offender **202** has not begun to blow, a sound, such as a beep, and/or a tactile output, such as a vibration are output from RBAM **200**. The sound and/or vibration are repeated periodically, such as every two minutes, until T_{grace} expires or Offender **202** begins to blow. A short time after Offender **202** begins to blow, Message **602** changes to "STOP" and is displayed on OLED Display **11**. When RBAM **200** determines that a valid sample has been received, Message **602** then changes to "ANALYZING DATA." Message **602** then changes to "TEST COMPLETE." Message **602** then changes to "SHUTTING DOWN" and OLED Display **11** goes dark when shut down is complete.

If RBAM **200** determines that an invalid sample has been received, Message **602** changes to "BLOW STEADY" and is displayed on OLED Display **11** (block **332** in **FIG. 3B**). In addition, a sound and/or vibration may be output to Offender **202**.

If the BrAC measured is greater than the LLOD, the Tᵣₑₜₑₛₜ period of time must pass before a retest can be performed. If there is a negative facial match upon taking the breath test, a second test can be taken right away without having to wait for the Tᵣₑₜₑₛₜ period of time to pass. Typically, RBAM **200** will not output any reasons to Offender **202** as to the nature of the failure that requires a retest (*i.e.*, negative facial match, BrAC limit exceeded, etc.). If a retest is required, Message **602** is changed to "RETEST IN 3 MIN" (where Tᵣₑₜₑₛₜ is three minutes) and is displayed on OLED Display **11** (block **350** in **FIG. 3B**). One minute later, Message **602** is changed to "RETEST IN 2 MIN." One minute later, Message **602** is changed to "RETEST IN 1 MIN." When it is time to begin the test, Message **602** will change to "BLOW' (block **354** in **FIG. 3C**) and is displayed on OLED Display **11** and the sequence above is repeated.

If a valid sample is not received, then Message **602** will change to "BLOW STEADY" (block **370** in **FIG. 3C**) and is displayed on OLED Display **11** and the sequence above is repeated. In addition, a sound and/or vibration may be output to Offender **202**. RBAM **200** will upload the test results to Monitor Network **206**, and then turn itself off.

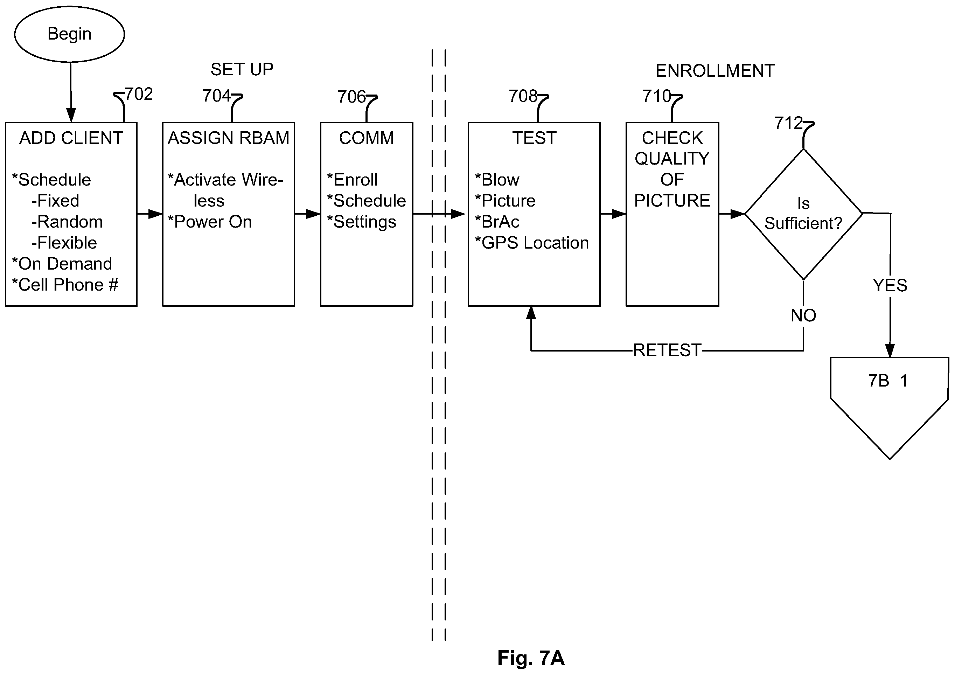

**Figures 7A-7D** show a flow chart of a general method of implementation in an embodiment of remote breath alcohol monitoring. Referring now to **FIGS. 7A-7D**, the set up process begins in block **702**, typically at Supervising Agency **210**. Supervising Agency **210** may be a court, government agency, law enforcement agency, or private corrections service provider working in conjunction with a local judicial district to handle a variety of offenders associated with DUI/DWI, probation, specialty courts, family courts, and underage drinking. In block **702** Supervising Agency **210** adds a new client, such as Offender **202**, through the website available over the Internet **216** at Monitor Network **206**. Based upon the circumstances and/or court orders associated with Offender **202**, a schedule for taking breath tests is established, which may be a fixed schedule, a random schedule, a flexible schedule, or any combination of the above. A fixed schedule would require a test at a fixed time, such as 8:00 am on a particular day. For a random test schedule, personnel at Supervising Agency **210** through the user interface for the website for setting up a new client select a window of time during the day, such as three hours as a default time, and a random time during that three hour block will be generated for taking a breath test. For a flexible schedule, a window of time is selected (*e*.*g*., 10:00 am to 1:00 pm), and Offender **202** can provide a breath test any time within that window. The schedule may also be provisioned to receive on-demand breath tests. The cell phone number of Offender **202** is also associated with the schedule. When a scheduled test is eminent, a text message or cell phone call can be made to the cell phone number of Offender **202** as a courtesy reminder to Offender **202** that there is an upcoming breath test. In another embodiment, Offender **202** may turn RBAM **200** on whenever they want and take a breath test, either instead of a test schedule, or in addition to a test schedule.

In block **704** an RBAM **200** is assigned to Offender **202**. The serial number of the RBAM **200** is associated with the name of Offender **202**. The wireless capability of the RBAM **200** is activated on the wireless network through a wireless carrier or intermediate wireless service provider, and RBAM **200** is powered on. In block **706** the assigned RBAM **200** communicates with Monitor Network **206** and the schedule and other settings that were established for Offender **202** are downloaded from Monitor Network **206** to RBAM **200**. A supervisory person or agent associated with Supervising Agency **210** could also perform the set up process from a laptop in the field as long as a connection to Internet **216** is available. Alternatively, RBAM **200** can simply be mailed to Offender **202** who powers it on and as long as a connection to Cellular Network **204** is available, the schedule and other settings for Offender **202** may be downloaded from Monitor Network **206** to RBAM **200**.

The enrollment process begins in block **708** where Offender **202** will take a breath test. Offender **202** may first be required or offered the opportunity to review some training materials (printed matter, videos, etc.) on how to use RBAM **200**. Offender **202** blows through Breath Tube **14**, RBAM **200** takes an enrollment image of Offender **202**. An enrollment template is extracted from the enrollment image and that enrollment template will be used as a comparison for future tests. The template is a mathematical model that reflects the characteristics of the facial image. There are critical factors/numbers when performing a facial match, such as the distance between the pupils (typically the most critical feature), the shape of the face and facial features, the location of the nose and mouth with respect to each other and the pupils, the shape of the eyes, etc. The template defines these critical factors/numbers. Every image taken during a breath test has a template extracted from the image, and that template is compared to the enrollment template for facial matching. The location fix where the test was taken is also captured for the breath test. The facial matching software processes the image taken in block **710** and generates a quality score for the image. Decision block **712** determines if the quality score is greater than or equal to a predetermined image quality score deemed to be of high enough quality to serve to extract a template for future facial matching purposes. If not, output is sent to OLED Display **11** or to Monitor Network **206** requesting to take another test so that another image can be taken. Once the facial matching software determines that a sufficiently high quality image has been taken, it extracts a template that is stored on RBAM **200** to be used for facial matching of future tests provided by Offender **202**. Next, in block **714** (*see* **FIG**. **7B**) the enrollment image is uploaded to Monitor Network **206** which stores the enrollment image associated with Offender **202**. This enrollment image is also typically used for human comparison if needed, and a lower resolution version of this image is often all that is required to do so. Other embodiments may upload enrollment images to the Monitor Network **206** and have Monitor Network **206** generate quality scores, do the template extraction, and store the enrollment template. Additional embodiments may perform some of these functions on RBAM **200** and others on Monitor Network**206**, or even perform them in both places.

The training process in block **716** enables Offender **202** to practice holding RBAM **200** properly, lining up RBAM **200** properly with the eyes or nose as reflected in Mirrors **17**, and practice blowing so Offender **202** can learn to not blow too hard but also not blow too softly. When Offender **202** is finished with training, by holding down the Power On/Off Switch **21** for five seconds, or after five minutes of inactivity, the training mode will stop.

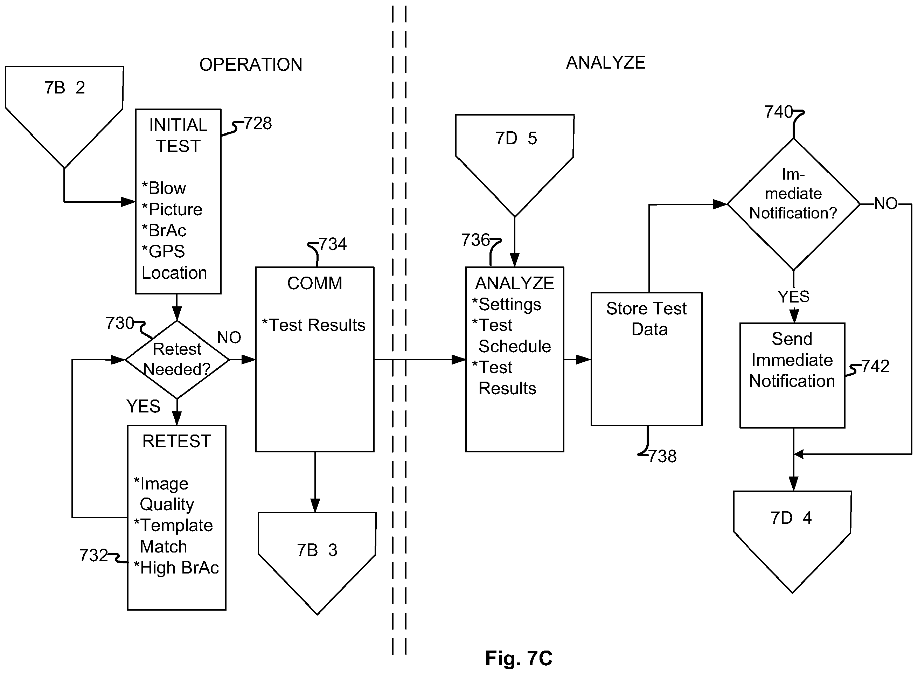

The normal operation process begins in block **718** where RBAM **200**, in one embodiment, checks in every twenty minutes with Monitor Network **206** to see if any changes to the settings, the schedule, or if an on-demand test has been ordered. The check-in interval is chosen based on trade-offs between immediacy of pulling new data down to RBAM **200** and power consumption, and any interval could be used. Changes are uploaded to RBAM **200** in block **720**. When it is time for a scheduled test in block **722**, a prompt may be sent in block **724** to Offender **202** via a text message or a phone call with a pre-recorded message to the cell phone associated with Offender **202**. In block **726**, when the scheduled time arrives, RBAM **200** turns on and sends a prompt to Offender **202** to begin blowing into Breath Tube **14** of RBAM **200** as described above in more detail in reference to **FIG. 6**. In block **728** (*see* **FIG. 7C**) Offender **202** blows into Breath Tube **14** inserted into RBAM **200**, a still image is taken by Camera **18**, the breath sample is processed by Fuel Cell Assembly With Tubing **10**, and the location fix is obtained. The facial recognition software analyzes the facial image and determines a quality score for the image and creates a template for this test. If the quality score of the image does not meet a threshold value, no facial recognition attempt will be made. The template for this test is compared to the template extracted from the enrollment image taken in block **708** and stored in memory in Processor Circuit Board Assembly **8**, and a match score is determined. The match score must meet certain predefined criteria to be considered a facial match. In one embodiment the match score must be above a predefined threshold value to be considered a facial match. Raising the acceptable threshold value would force a stricter match, and lowering the threshold value would loosen the criteria to declare a match. The goal is to set the predetermined criteria to minimize false rejections when Offender **202** did indeed take the test, and also minimize false acceptance of a match when Offender **202** did not take the test. In one embodiment of the invention, the image taken by Camera **18** is uploaded to Monitor Network **206** and facial matching is done at Monitor Network **206**, or, just the template is uploaded to be compared with the enrollment template stored at Monitor Network **206**, or any combination. In another embodiment, when Offender **202** begins blowing, prior to taking the image required for facial matching, Camera **18** first begins taking a video or series of still images of Offender **202** to be analyzed by a liveness detection component of the facial recognition software, which analyzes the video or series of images to determine movement of the eyes, eyebrows, nose, mouth, etc. A printed photograph or mask placed in front of RBAM **200** will not have any movement of these facial parts.

In decision block **730**, based upon the results of the breath test, facial match result, or quality score of the image taken, RBAM **200** may output a message through OLED Display **11** to Offender **202** to retake the breath test in block **732**. A retest is typically required for a positive breath test and may be required for a poor quality score or negative facial match. The retest is evaluated again in decision block **730**. If there is a facial match, and the quality score of the image is acceptable, and a negative breath test, RBAM **200** in block **734** will send to Monitor Network **206** the results of both tests (BrAC; location fix; and images, match score, templates, and the quality scores from the facial recognition analysis). (*See* **FIGS. 3A-3D** for details on the different test results that may be sent to Monitor Network **206**.)

The analyze operation begins in block **736** where Monitor Network **206** will analyze the test results received: a single test; or, a test and a retest that are combined into a single test event. The outcome of the initial test may be one of the following: missed, incomplete, passed, or failed along with a positive facial match or a negative facial match, and the location fix. For a missed test, Offender **202** never attempted to blow into RBAM **200**. An incomplete test is the result of Offender **202** attempting to blow but failing to deliver a valid sample. This may be the result of Offender **202** not blowing hard enough, or not blowing long enough, or some other reason that results in the failed delivery of a valid sample. For a valid sample, the result is either passed or failed based upon the BrAC level. A positive facial match indicates that the person delivering the sample is the Offender **202** enrolled with the RBAM **200**. A negative facial match may be the result of a different person delivering the valid sample. Or, even if Offender **202** delivered the valid sample, a negative facial match may result due to Offender **202** wearing sunglasses, hair covering the face, or other obstruction such as a hat or scarf.

Monitor Network **206** will report a single missed test result as "Missed"; a single incomplete test result as "Incomplete"; a single passed test with a positive facial match as "Passed"; and a single passed test with a negative facial match as "Needs Review". Needs Review test results may be set to be an immediate notification to Supervising Agency **210**.

A failed test is automatically followed by a confirmation test. The possible outcomes for a confirmation test are the same as for an initial test. The confirmation test is the opportunity afforded to Offender **202** to prove that the failed first test was mouth alcohol (such as mouthwash) and not a result of the consumption of alcohol. In the embodiment in which facial matching is performed on RBAM **200**, a retest may also be required for a negative facial match.

For a combined test (failed initial test followed by a confirmation test), Monitor Network **206** will report a missed confirmation test as "Failed" (a positive initial test followed by a missed confirmation test (*see* Block **362** in FIG. 3D)). Offender **202** had the opportunity to take the confirmation test, but did not. Monitor Network **206** will report a missed confirmation test as "Failed". Monitor Network **206** will report an incomplete confirmation test as "Failed" (a positive initial test followed by an incomplete confirmation test (see Block **380** in FIG. 3D)). Offender **202** had the opportunity to take the confirmation test, but failed to deliver a valid sample. Monitor Network **206** will report a negative confirmation test as "Passed" (a positive initial test followed by a negative confirmation test (*see* Block **386** in FIG. 3D)). Monitor Network **206** will report a positive but abnormal confirmation test as "Failed" (a positive initial test followed by a positive but abnormal confirmation test (*see* Block **392** in FIG. 3D)). Monitor Network **206** will report a positive confirmation test as "Failed" (a positive initial test followed by a positive confirmation test (*see* Block **396** in FIG. 3D)). In this scenario, the delta between the two tests is less than or equal to 0.02%.

Location fix is determined with each test. In one embodiment this is accomplished by obtaining the GPS coordinates, which are obtained using a GPS receiver. The GPS receiver may be a stand-alone component within the RBAM, or may be built into the cellular module. However, in some cases, typically if Offender **202** is indoors, the satellite signal needed to get the GPS coordinates cannot be obtained and several secondary methods may be used. In one embodiment those secondary methods include cell tower triangulation by RBAM **200** and the cellular service provider. If this option is not available, then in one embodiment, location fix is determined by the nearest cell site.

Different types of test results can be sent on an immediate or priority notification basis using Communication Link **222** if Supervising Agency **210** elects to be so notified. For example, Supervising Agency **210** can elect to be notified immediately for failed tests, missed tests, incomplete tests, and needs review. Even if immediate notification is not chosen, all test results are uploaded to Monitor Network **206** upon completion of the test and stored and are immediately available for review by Supervising Agency **210** via the website. An agent at Supervising Agency **210** may review the uploaded data in different ways. For all Offenders **202** being monitored, all their test results can be displayed in the order the tests were uploaded, with the oldest test results on the top of the list, or, with the newest test results on the top of the list. The list can also be sorted by individual Offender **202**, oldest tests on top or newest tests on top.

For an agency level view, only alerts and exceptions may be displayed. This exception based monitoring makes more efficient use of agent's time by focusing agent attention on the tests that matter. All failed, missed, incomplete, and needs review test results can be displayed for the agent, and as the agent deals with each one, the action taken can be logged and the alert or exception resolved. Alerts can include no communication by RBAM **200** with Monitor Network **206** for a given period of time, tamper alerts, and housing breach alerts. Without facial matching, prior art systems had to report everything to a supervising agency and every single one of the tests needed to be looked at and evaluated. Facial matching coupled with automatic confirmation testing eliminates a lot of data that no longer needs to be reviewed by an agent at Supervising Agency **210**. This exception based reporting as disclosed herein is a tremendous improvement over existing remote breath monitoring systems. In addition, when Offender **202** is confronted with a failed test, the date and time, the BrAC, their facial image, and their location fix, it makes it very difficult for Offender **202** to deny what they did.

Immediate notification may be accomplished in the following ways: by text message; by email; or by a page sent to a pager. Supervising Agency **210** can then take whatever action they desire. An agent for Supervising Agency **210** may call Offender **202** and inquire as to why Offender **202** missed taking the test. For a missed test, Supervising Agency **210** may have information that Monitor Network **206** does not. For example, Offender **202** may have called in and said they could not take the test for whatever reason.

Upon notification of a missed test, or for some other reason, Supervising Agency **210** may request an on-demand test. An agent for Supervising Agency **210** can click a button on their web application ordering an on-demand test for Offender **202**. Since RBAM **200** checks in with Monitor Network **206** periodically, RBAM **200** will receive the on-demand test order within its check-in interval, wake up, and prompt Offender **202** to take a test. Monitor Network **206** may also send a text message to Offender **202,** or the agent may call Offender **202** immediately after the on-demand test is ordered in the web application. The text message or agent will then instruct Offender **202** to hold down Power On/Off Switch **21** for five seconds, which causes RBAM **200** to power up and communicate with Monitor Network **206**, receive the on-demand test order, and prompt Offender **202** to take a test.

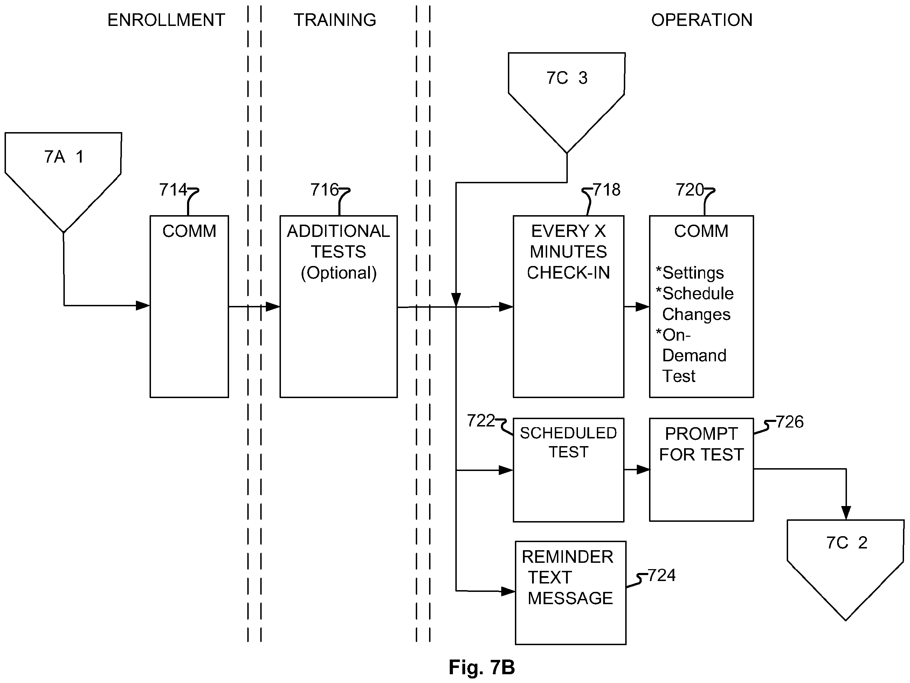

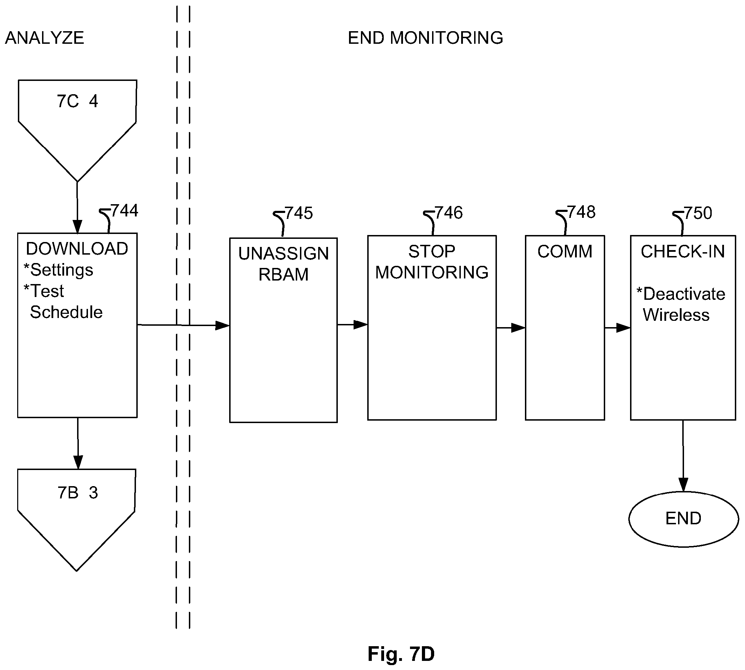

In block **738** the test results are stored and are immediately available for review by an agent at Supervising Agency **210**. Decision block **740** determines if an immediate or priority notification is necessary based upon preferences established by Supervising Agency **210**. If yes, then in block **742** notification is sent from Monitor Network **206** to Supervising Agency **210** or to a specific agent or supervisor associated with Supervising Agency **210** via communication link **222**. Control returns to block **736** to await the next test results needing analysis. If immediate notification is not needed, then in block **744** (see **FIG. 7D**) if there are any changes to the settings or schedule, those changes are downloaded to RBAM **200**. RBAM **200** will then turn off and control returns to blocks **718** (check in every fifteen to twenty minutes), **722** (wait for next scheduled test), and **724** (send reminder text message) (*see* **FIG. 7B**).

When Offender **202** no longer needs to be monitored as determined by Supervising Agency **210**, in block **745** RBAM **200** is unassigned from Offender **202**. In block **746** monitoring is stopped and Offender **202** returns RBAM **200** to Supervising Agency **210**. In block **748** Monitor Network **206** will communicate with RBAM **200** and in block **750** check RBAM **200** back into inventory, where it becomes available to the next Offender **202**, and RBAM **200** is deactivated from the wireless network. After RBAM **200** is deactivated from the wireless network the method ends.

## Claims

1. A remote breath alcohol monitor configured for capturing a facial image of an offender while the offender is delivering a breath sample, and comprising:
a vent (25) opening in the remote breath alcohol monitor disposed for receiving a breath sample in an ethanol sensor within the remote breath alcohol monitor; at least one circuit board assembly (7) within the remote breath alcohol monitor arranged for processing the output of the ethanol sensor to determine a breath alcohol content;
a camera (18) connectable to the at least one circuit board assembly (7) arranged for capturing a facial image of a user;
a wireless cellular phone module embedded in the at least one circuit board assembly (7) and arranged for sending the breath alcohol content and the facial image;
the at least one circuit board assembly (7) being arranged to perform a facial match on the facial image compared to an enrollment image, and
a breath tube (14) connectable to the vent (25)
**characterized by**:
a pressure transducer within a fuel cell assembly with tubing (10) arranged to measure that the offender has blown hard enough and long enough for the breath sample to be valid;
in that the breath tube (14) is flat in cross-section thus to prevent insertion of a smaller round tube thereinto and preventing a user from tilting his/her head too much from side-to-side, that is, no more than about 10°;
and in that the length of the breath tube (14) is such as to achieve a focal length between the camera and the user's face.

2. A remote breath alcohol monitor as claimed in claim 1 and wherein the wireless cellular phone module is arranged for sending a breath alcohol content signal and a facial image signal to a monitor network.

3. A remote breath alcohol monitor as claimed in claim 1 or claim 2 further comprising a GPS receiver connectable to the at least one circuit board assembly (7) arranged for capturing a location fix in conjunction with receiving the breath sample, the phone module being also arranged to transmit a location signal to the monitor network.

4. The remote breath alcohol monitor of claim 3 further comprising:
a secondary means to acquire the location fix when a satellite signal needed for the GPS receiver is not available, the secondary method comprising at least one of:
a cell tower triangulation; a nearest cell site; and a location based service.

5. The remote breath alcohol monitor of any one of the preceding claims further comprising a memory connectable to the at least one circuit board assembly (7) and arranged for storing the breath alcohol content, the facial image and, where provided, the location fix.

6. The remote breath alcohol monitor of any one of the preceding claims and wherein the camera (18) is arranged to operate in a liveness face detection mode.

7. The remote breath alcohol monitor of any one of the preceding claims and arranged to signal to a user that a breath test is required.

8. The remote breath alcohol monitor as claimed in any one of the preceding claims and incorporating a display (11).

9. The remote breath alcohol monitor as claimed in any one of the preceding claims and incorporating a mirror (17) arranged for assisting in properly aligning a user's face with the camera (18).

10. A method for remote breath alcohol monitoring, the method comprising the steps of:
(a) receiving a first breath sample in a remote breath alcohol monitor, the remote breath alcohol monitor being as claimed in claim 1;
(b) analyzing by the remote breath alcohol monitor the first breath sample for a first breath alcohol content;
(c) capturing with a camera (18) in the remote breath alcohol monitor a first facial image;
(d) performing a first facial match on the first facial image compared to an enrollment image; and
(e) sending by the remote breath alcohol monitor the first breath alcohol content and the first facial image to a monitor network.

11. The method according to claim 10 further comprising the steps of:
capturing a first location fix in conjunction with receiving the first breath sample; and
sending the first location fix to the monitor network along with the first breath alcohol content and the first facial image.

12. The method according to claim 10 or claim 11 further comprising the steps of:
when the first breath alcohol content is greater than or equal to a predetermined value, performing the following steps in place of step (e):
(f) generating by the remote breath alcohol monitor an output requesting a confirmation test;
(g) receiving a second breath sample in the remote breath alcohol monitor;
(h) analyzing by the remote breath alcohol monitor the second breath sample for a second breath alcohol content;
(i) capturing with the camera in the remote breath alcohol monitor a second facial image;
(j) performing a second facial match on the second facial image compared to the enrollment image; and
(k) sending by the remote breath alcohol monitor the first breath alcohol content and the first facial image, which comprise an initial test, and the second breath alcohol content and the second facial image, which comprise the confirmation test, to the monitor network.

13. The method according to claim 12 further comprising the steps of:
downloading from the monitor network an on-demand breath test request;
generating by the remote breath alcohol monitor an output requesting a breath test; and
repeating steps (a) through (k).

14. The method according to any one of claims 10 to 13 further comprising the steps of:
managing power consumption in the remote breath alcohol monitor by:
running a low power processor continuously to control a first set of functions; and
running a high power processor only as needed to control a second set of functions.

15. A method for monitoring a plurality of offenders for alcohol consumption, the method comprising the steps set out in any one of claims 10 to 14 and further, of:
(a) storing in at least one server in a monitor network information on a plurality of offenders, each of which has been assigned a remote breath alcohol monitor;
(b) storing in the at least one server information on a plurality of supervising agencies, each of which is responsible for monitoring a subset of the plurality of offenders;
(c) receiving in the at least one server a plurality of test results captured by the plurality of remote breath alcohol monitors, wherein each of the plurality of test results comprises at least one breath alcohol content and at least one facial image;
(d) determining by one or more rules stored in the at least one server if any of the plurality of test results requires an immediate notification to be sent; and
(e) sending an alert to the supervising agency responsible for each of the plurality of offenders whose test results require an immediate notification.

## Patentansprüche

1. Atemalkohol-Fernüberwachungsvorrichtung, die konfiguriert ist zum Aufnehmen eines Gesichtsbilds eines Straftäters, während der Straftäter eine Atemprobe abgibt, umfassend:
eine Öffnung (25), die sich in der Atemalkohol-Fernüberwachungsvorrichtung öffnet und angeordnet ist für das Empfangen einer Atemprobe in einem Ethanolsensor in der Atemalkohol-Fernüberwachungsvorrichtung,
wenigstens eine Leiterplattenanordnung (7) in der Atemalkohol-Fernüberwachungsvorrichtung, die angeordnet ist zum Verarbeiten der Ausgabe des Ethanolsensors für das Bestimmen eines Atemalkoholgehalts,
eine Kamera (18), die mit der wenigstens einen Leiterplattenanordnung (7) verbunden werden kann und angeordnet ist zum Aufnehmen eines Gesichtsbilds des Straftäters,
ein Mobilfunkmodul, das in der wenigstens einen Leiterplattenanordnung (7) eingebettet ist und angeordnet ist zum Senden des Atemalkoholgehalts und des Gesichtsbilds,
wobei die wenigstens eine Leiterplattenanordnung (7) angeordnet ist zum Durchführen eines Gesichtsabgleichs auf dem Gesichtsbild für einen Vergleich mit einem Registrierungsbild, und
ein Atemrohr (14), das mit der Öffnung (25) verbunden werden kann,
**gekennzeichnet durch**:
einen Druckaufnehmer in einer Brennstoffzellenanordnung mit einer entsprechenden Rohrleitung (10), der angeordnet ist zum Messen, ob der Straftäter ausreichend fest und ausreichend lang für eine gültige Atemprobe geblasen hat,
wobei das Atemrohr (14) einen flachen Querschnitt aufweist, um das Einstecken eines kleineren runden Rohrs zu verhindern und um zu verhindern, dass der Straftäter seinen Kopf zu weit, d.h. mehr als 10°, zu einer Seite neigt, und
wobei die Länge des Atemrohrs (14) derart gewählt ist, dass eine Brennweite zwischen der Kamera und dem Gesicht des Straftäters erhalten wird.

2. Atemalkohol-Fernüberwachungsvorrichtung nach Anspruch 1, wobei das Mobilfunkmodul angeordnet ist zum Senden eines Atemalkoholgehaltsignals und eines Gesichtsbildsignals an ein Überwachungsnetz.

3. Atemalkohol-Fernüberwachungsvorrichtung nach Anspruch 1 oder 2, die weiterhin einen GPS-Empfänger umfasst, der mit der wenigstens einen Leiterplattenanordnung (7) verbunden werden kann und angeordnet ist zum Erfassen des Standorts in Verbindung mit dem Empfang der Atemprobe, wobei das Mobilfunkmodul auch angeordnet ist zum Senden eines Standortsignals an das Überwachungsnetz.

4. Atemalkohol-Fernüberwachungsvorrichtung nach Anspruch 3, das weiterhin umfasst:
eine sekundäre Einrichtung zum Erfassen des Standorts, wenn ein für den GPS-Empfänger erforderliches Satellitensignal nicht verfügbar ist, wobei die sekundäre Einrichtung wenigstens eine der folgenden Methoden verwendet: eine Mobilfunkmast-Triangulation, einen nächsten Mobilfunkmast und einen standortbezogenen Dienst.

5. Atemalkohol-Fernüberwachungsvorrichtung nach einem der vorstehenden Ansprüche, die weiterhin einen Speicher umfasst, der mit der wenigstens einen Leiterplattenanordnung (7) verbunden werden kann und angeordnet ist zum Speichern des Atemalkoholgehalts, des Gesichtsbilds und, sofern vorgesehen, des Standorts.

6. Atemalkohol-Fernüberwachungsvorrichtung nach einem der vorstehenden Ansprüche, wobei die Kamera (18) angeordnet ist für einen Betrieb in einem die Lebendigkeit erkennenden Gesichtserkennungsmodus.

7. Atemalkohol-Fernüberwachungsvorrichtung nach einem der vorstehenden Ansprüche, die angeordnet ist zum Signalisieren für einen Straftäter, dass ein Atemtest erforderlich ist.

8. Atemalkohol-Fernüberwachungsvorrichtung nach einem der vorstehenden Ansprüche, die ein Display (11) umfasst.

9. Atemalkohol-Fernüberwachungsvorrichtung nach einem der vorstehenden Ansprüche, die einen Spiegel (17) umfasst, der angeordnet ist, um bei der korrekten Ausrichtung des Gesichts des Straftäters mit der Kamera (18) zu helfen.

10. Verfahren für eine Atemalkohol-Fernüberwachung, wobei das Verfahren die folgenden Schritte umfasst:
(a) Empfangen einer ersten Atemprobe in einer Atemalkohol-Fernüberwachungsvorrichtung gemäß dem Anspruch 1,
(b) Analysieren, mittels der Atemalkohol-Fernüberwachungsvorrichtung, der ersten Atemprobe auf einen ersten Atemalkoholgehalt,
(c) Aufnehmen, mit einer Kamera (18) in der Atemalkohol-Fernüberwachungsvorrichtung, eines ersten Gesichtsbilds,
(d) Durchführen eines ersten Gesichtsabgleichs auf dem ersten Gesichtsbild für einen Vergleich mit einem Registrierungsbild, und
(e) Senden, durch die Atemalkohol-Fernüberwachungsvorrichtung, des ersten Atemalkoholgehalts und des ersten Gesichtsbilds an ein Überwachungsnetz.

11. Verfahren nach Anspruch 10, das weiterhin die folgenden Schritte umfasst:
Erfassen eines ersten Standorts in Verbindung mit dem Empfangen der ersten Atemprobe, und
Senden des ersten Standorts an das Überwachungsnetz zusammen mit dem ersten Atemalkoholgehalt und dem ersten Gesichtsbild.

12. Verfahren nach Anspruch 10 oder 11, das weiterhin die folgenden Schritte umfasst:
wenn der erste Atemalkoholgehalt größer als oder gleich einem vorbestimmten Wert ist, Durchführen der folgenden Schritte anstelle des Schritts (e):
(f) Erzeugen, durch die Atemalkohol-Fernüberwachungsvorrichtung, einer Ausgabe, die einen Bestätigungstest anfordert,
(g) Empfangen einer zweiten Atemprobe in der Atemalkohol-Fernüberwachungsvorrichtung,
(h) Analysieren, durch die Atemalkohol-Fernüberwachungsvorrichtung, der zweiten Atemprobe auf einen zweiten Atemalkoholgehalt,
(i) Aufnehmen, mit der Kamera in der Atemalkohol-Fernüberwachungsvorrichtung, eines zweiten Gesichtsbilds,
(j) Durchführen eines zweiten Gesichtsabgleichs auf dem zweiten Gesichtsbild für einen Vergleich mit dem Registrierungsbild, und
(k) Senden, durch die Atemalkohol-Fernüberwachungsvorrichtung, des ersten Atemalkoholgehalts und des ersten Gesichtsbilds eines anfänglichen Tests und des zweiten Atemalkoholgehalts und des zweiten Gesichtsbilds des Bestätigungstests an das Überwachungsnetz.

13. Verfahren nach Anspruch 12, das weiterhin die folgenden Schritte umfasst:
Herunterladen, von dem Überwachungsnetz, einer bei Bedarf gestellten Atemtestanforderung,
Erzeugen, durch die Atemalkohol-Fernüberwachungsvorrichtung, einer Ausgabe, die einen Atemtest anfordert, und
Wiederholen der Schritte (a) bis (k).

14. Verfahren nach einem der Ansprüche 10 bis 13, das weiterhin die folgenden Schritte umfasst:
Verwalten des Stromverbrauchs in der Atemalkohol-Fernüberwachungsvorrichtung durch:
Ausführen eines Niedrigleistung-Prozessors kontinuierlich für das Steuern eines ersten Satzes von Funktionen, und
Ausführen eines Hochleistung-Prozessors nur bei Bedarf für das Steuern eines zweiten Satzes von Funktionen.

15. Verfahren zum Überwachen einer Vielzahl von Straftätern auf Alkoholkonsum, wobei das Verfahren die Schritte gemäß den Ansprüchen 10 bis 14 und weiterhin die folgenden Schritte umfasst:
(a) Speichern, in wenigstens einem Server in einem Überwachungsnetz, von Informationen zu einer Vielzahl von Straftätern, denen jeweils eine Atemalkohol-Fernüberwachungsvorrichtung zugewiesen wurde,
(b) Speichern, in dem wenigstens einen Server, von Informationen zu einer Vielzahl von Aufsichtsinstanzen, die jeweils für das Überwachen eines Teils aus der Vielzahl von Straftätern verantwortlich sind,
(c) Empfangen, in dem wenigstens einen Server, einer Vielzahl von durch die Vielzahl von Atemalkohol-Fernüberwachungsvorrichtungen erfassten Testergebnissen, wobei jeder aus der Vielzahl von Testergebnissen wenigstens einen Atemalkoholgehalt und wenigstens ein Gesichtsbild umfasst,
(d) Bestimmen, anhand von einer oder mehreren in dem wenigstens einen Server gespeicherten Regeln, ob eines aus der Vielzahl von Testergebnissen das unmittelbare Senden einer Benachrichtigung erfordert, und
(e) Senden einer Warnung an die Aufsichtsinstanz, die für einen aus der Vielzahl von Straftätern, dessen Testergebnisse eine unmittelbare Benachrichtigung erfordern, verantwortlich ist.

## Revendications

1. Dispositif de contrôle à distance d'alcoolémie configuré pour capturer une image faciale d'un contrevenant pendant que le contrevenant délivre un échantillon d'haleine, et comprenant :
un évent (25) s'ouvrant dans le dispositif de contrôle à distance d'alcoolémie disposé pour recevoir un échantillon d'haleine dans un capteur d'éthanol au sein du dispositif de contrôle à distance d'alcoolémie ; au moins un ensemble de carte de circuit imprimé (7) au sein du dispositif de contrôle à distance d'alcoolémie agencé pour traiter la sortie du capteur d'éthanol afin de déterminer un taux d'alcoolémie ;
une caméra (18) pouvant être raccordée à l'au moins un ensemble de carte de circuit imprimé (7) agencée pour capturer une image faciale d'un utilisateur ;
un module de téléphone cellulaire sans fil intégré dans l'au moins un ensemble de carte de circuit imprimé (7) et agencé pour envoyer le taux d'alcoolémie et l'image faciale ;
l'au moins un ensemble de carte de circuit imprimé (7) étant agencé pour réaliser une concordance faciale sur l'image faciale en comparaison à une image d'enrôlement, et
un tube respiratoire (14) pouvant être raccordé à l'évent (25)
**caractérisé en ce que** :
un transducteur de pression au sein d'un ensemble de piles à combustible avec une tubulure (10) est agencé pour mesurer le fait que le contrevenant a soufflé suffisamment fort et suffisamment longtemps pour que l'échantillon d'haleine soit valide ;
**en ce que** le tube respiratoire (14) a une section plate pour empêcher ainsi l'insertion d'un tube rond plus petit à l'intérieur et empêcher un utilisateur de trop pencher la tête d'un côté à l'autre, c'est-à-dire, de pas plus de 10° ;
et **en ce que** la longueur du tube respiratoire (14) est de nature à atteindre une longueur focale entre la caméra et le visage de l'utilisateur.

2. Dispositif de contrôle à distance d'alcoolémie selon la revendication 1 et dans lequel le module de téléphone cellulaire sans fil est agencé pour envoyer un signal de taux d'alcoolémie et un signal d'image faciale à un réseau de contrôle.

3. Dispositif de contrôle à distance d'alcoolémie selon la revendication 1 ou la revendication 2, comprenant en outre un récepteur GPS pouvant être raccordé à l'au moins un ensemble de carte de circuit imprimé (7) agencé pour capturer un repère de localisation conjointement à la réception de l'échantillon d'haleine, le module de téléphone étant également agencé pour transmettre un signal de localisation au réseau de contrôle.

4. Dispositif de contrôle à distance d'alcoolémie selon la revendication 3 comprenant en outre :
un moyen secondaire pour acquérir le repère de localisation lorsqu'un signal satellite dont le récepteur GPS a besoin n'est pas disponible, le procédé secondaire comprenant au moins un parmi : une triangulation de tour de téléphonie cellulaire ; un site cellulaire le plus proche ; et un service basé sur la géolocalisation.

5. Dispositif de contrôle à distance d'alcoolémie selon l'une quelconque des revendications précédentes comprenant en outre une mémoire pouvant être raccordée à l'au moins un ensemble de carte de circuit imprimé (7) et agencée pour stocker le taux d'alcoolémie, l'image faciale et, le cas échéant, le repère de localisation.

6. Dispositif de contrôle à distance d'alcoolémie selon l'une quelconque des revendications précédentes et dans lequel la caméra (18) est agencée pour fonctionner dans un mode de détection de visage en direct.

7. Dispositif de contrôle à distance d'alcoolémie selon l'une quelconque des revendications précédentes et agencé pour signaler à un utilisateur qu'une épreuve respiratoire est exigée.

8. Dispositif de contrôle à distance d'alcoolémie selon l'une quelconque des revendications précédentes et incorporant un afficheur (11).

9. Dispositif de contrôle à distance d'alcoolémie selon l'une quelconque des revendications précédentes et incorporant un miroir (17) agencé pour aider à aligner correctement le visage d'un utilisateur avec la caméra (18).

10. Procédé de contrôle à distance d'alcoolémie, le procédé comprenant les étapes de :
(a) réception d'un premier échantillon d'haleine dans un dispositif de contrôle à distance d'alcoolémie, le dispositif de contrôle à distance d'alcoolémie étant tel que revendiqué à la revendication 1 ;
(b) analyse par le dispositif de contrôle à distance d'alcoolémie du premier échantillon d'haleine pour avoir un premier taux d'alcoolémie ;
(c) capture avec une caméra (18) dans le dispositif de contrôle à distance d'alcoolémie d'une première image faciale ;
(d) réalisation d'une première concordance faciale sur la première image faciale en comparaison à une image d'enrôlement ; et
(e) envoi par le dispositif de contrôle à distance d'alcoolémie du premier taux d'alcoolémie et de la première image faciale à un réseau de contrôle.

11. Procédé selon la revendication 10, comprenant en outre les étapes de :
capture d'un premier repère de localisation conjointement à la réception du premier échantillon d'haleine ; et
envoi du premier repère de localisation au réseau de contrôle ainsi que du premier taux d'alcoolémie et de la première image faciale.

12. Procédé selon la revendication 10 ou la revendication 11 comprenant en outre les étapes de :
lorsque le premier taux d'alcoolémie est supérieur ou égal à une valeur prédéterminée, réalisation des étapes suivantes à la place de l'étape (e) :
(f) génération par le dispositif de contrôle à distance d'alcoolémie d'une sortie requérant une épreuve de confirmation ;
(g) réception d'un second échantillon d'haleine dans le dispositif de contrôle à distance d'alcoolémie ;
(h) analyse par le dispositif de contrôle à distance d'alcoolémie du second échantillon d'haleine pour avoir un second taux d'alcoolémie ;
(i) capture avec la caméra dans le dispositif de contrôle à distance d'alcoolémie d'une seconde image faciale ;
(j) réalisation d'une seconde concordance faciale sur la seconde image faciale en comparaison à l'image d'enrôlement ; et
(k) envoi par le dispositif de contrôle à distance d'alcoolémie du premier taux d'alcoolémie et de la première image faciale, qui comprennent une épreuve initiale, et du second taux d'alcoolémie et de la seconde image faciale, qui comprennent l'épreuve de confirmation, au réseau de contrôle.

13. Procédé selon la revendication 12, comprenant en outre les étapes de :
téléchargement depuis le réseau de contrôle d'une requête d'épreuve respiratoire à la demande ;
génération par le dispositif de contrôle à distance d'alcoolémie d'une sortie requérant une épreuve respiratoire ; et
répétition des étapes (a) à (k).

14. Procédé selon l'une quelconque des revendications 10 à 13 comprenant en outre les étapes de :
gestion de consommation d'énergie dans le dispositif de contrôle à distance d'alcoolémie par :
le fonctionnement en continu d'un processeur à faible consommation d'énergie pour commander un premier jeu de fonctions ; et
le fonctionnement d'un processeur à haute consommation d'énergie uniquement lorsque cela est nécessaire pour commander un second jeu de fonctions.

15. Procédé de contrôle d'une pluralité de contrevenants en matière de consommation d'alcool, le procédé comprenant les étapes présentées dans l'une quelconque des revendications 10 à 14 et, en outre, de :
(a) stockage, dans au moins un serveur dans un réseau de contrôle, d'informations sur une pluralité de contrevenants, dont chacun s'est vu attribuer un dispositif de contrôle à distance d'alcoolémie ;
(b) stockage, dans l'au moins un serveur, d'informations sur une pluralité d'agences de surveillance, dont chacune est responsable du contrôle d'un sous-ensemble de la pluralité de contrevenants ;
(c) réception, dans l'au moins un serveur, d'une pluralité de résultats d'épreuve capturés par la pluralité de dispositifs de contrôle à distance d'alcoolémie, dans lequel chacun de la pluralité de résultats d'épreuve comprend au moins un taux d'alcoolémie et au moins une image faciale ;
(d) détermination, par une ou plusieurs règles stockées dans l'au moins un serveur, du fait que l'un quelconque de la pluralité de résultats d'épreuve exige qu'une notification immédiate soit envoyée ; et
(e) envoi d'une alerte à l'agence de surveillance responsable de chacun de la pluralité de contrevenants dont les résultats d'épreuve exigent une notification immédiate.
